(19) Europäisches Patentamt
European Patent Office
Office européen des brevets

(11) **EP 4 156 179 A1**

(12) **EUROPÄISCHE PATENTANMELDUNG**

(43) Veröffentlichungstag:
**29.03.2023 Patentblatt 2023/13**

(21) Anmeldenummer: **21198655.9**

(22) Anmeldetag: **23.09.2021**

(51) Internationale Patentklassifikation (IPC):
**G10L 15/32** (2013.01)   **A61B 17/00** (2006.01)
**G10L 15/22** (2006.01)

(52) Gemeinsame Patentklassifikation (CPC):
**G10L 15/32; A61B 5/7267; A61B 5/749;**
A61B 5/0036; A61B 2017/00203; A61B 2505/05;
A61B 2562/0204; G10L 2015/223

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH 91052 Erlangen (DE)**

(72) Erfinder:
- **KUHRT, Sören 91056 Erlangen (DE)**
- **ROTHBAUER, Stefan 86156 Augsburg (DE)**
- **KILIAN, Lennart, Dr. 82131 Gauting (DE)**

(54) **SPRACHSTEUERUNG EINER MEDIZINISCHEN VORRICHTUNG**

(57) Verfahren zur Sprachsteuerung einer medizinischen Vorrichtung (1) mit den Schritten:
- Erfassen (S10) eines Audiosignals (E1) enthaltend eine auf die Steuerung der Vorrichtung gerichtete Spracheingabe einer Bedienperson;
- Analysieren (S20) des Audiosignals zum Bereitstellen eines ersten Sprachanalyseergebnisses (SAE1),
- Erkennen (S30) eines ersten Sprachbefehls (SSB1) der Bedienperson basierend auf dem ersten Sprachanalyseergebnis,
- Ermitteln (S50) eines Verifikationssignals (VS) für den ersten Sprachbefehl, umfassend:
- Analysieren (S51) des Audiosignals zum Bereitstellen eines zweiten Sprachanalyseergebnisses (SAE2),
- Erkennen (S52) eines zweiten Sprachbefehls (SSB2) der Bedienperson basierend auf dem zweiten Sprachanalyseergebnis,
- Vergleichen (S53) des ersten und des zweiten Sprachbefehls,
wobei das Verifikationssignal den ersten Sprachbefehl bestätigt, wenn der erste Sprachbefehl und der zweite Sprachbefehl ein Übereinstimmungskriterium (ÜK) erfüllen,

- Erzeugen (S60) eines Steuersignals (C1) zur Steuerung der medizinischen Vorrichtung basierend dem Verifikationssignal, sofern der erste Sprachbefehl anhand des Verifikationssignals bestätigt wurde, wobei das Steuersignal geeignet ist, die medizinische Vorrichtung entsprechend dem ersten Sprachbefehl zu steuern, und
- Eingabe (S70) des Steuersignals in die medizinische Vorrichtung.

FIG 3

**Beschreibung**

Die Erfindung betrifft ein Verfahren zur Sprachsteuerung einer medizinischen Vorrichtung durch Verarbeiten eines Audiosignals enthaltend eine auf die Steuerung der Vorrichtung gerichtete Spracheingabe einer Bedienperson. Insbesondere betrifft die Erfindung ein Verfahren zur erstfehlersicheren Sprachsteuerung einer medizinischen Vorrichtung. Die Erfindung betrifft auch ein entsprechendes medizinisches System aufweisend eine medizinische Vorrichtung.

Medizinische Vorrichtungen werden typischerweise zur Behandlung und/oder Untersuchung und/oder Überwachung eines Patienten eingesetzt, wie bspw. bildgebende Modalitäten wie Magnetresonanzvorrichtungen, Computertomographievorrichtungen, PET-Vorrichtungen (Positronen-Emissions-Tomographie-Vorrichtungen) oder Interventions- und/oder Therapievorrichtungen wie Strahlen- bzw. Radiotherapievorrichtungen. Die Behandlung und/oder Untersuchung des Patienten wird typischerweise von einem Bedienpersonal unterstützt.

Vor und während der Durchführung einer Behandlung und/oder Untersuchung eines Patienten mit einer solchen medizinischen Vorrichtung sind üblicherweise verschiedene Einstellungen an der medizinischen Vorrichtung vorzunehmen, wie beispielsweise eine Eingabe von Patientendaten, eine Einstellung verschiedener Geräteparameter und dergleichen. Diese Schritte werden von dem Bedienpersonal durchgeführt, wobei die Vornahme der Einstellungen der medizinischen Vorrichtung typischerweise über eine an der Vorrichtung bereitgestellte physische Benutzerschnittstelle erfolgt, in welche eine Bedienperson Eingaben vornehmen kann.

Um solche medizinischen Vorrichtungen wirtschaftlich zu betreiben, ist ein reibungsloser Arbeits- bzw. Verfahrensablauf wünschenswert. Insbesondere soll die Vornahme von Einstellungen so einfach wie möglich gestaltet werden. Dazu eignet sich insbesondere eine Sprachsteuerung, bei der eine Bedienperson Steuerbefehle über ein natürliches Sprachsignal an die medizinische Vorrichtung übergibt. Die DE 10 2006 045 719 B4 beschreibt in dieser Hinsicht ein medizinisches System mit Spracheingabevorrichtung, bei dem spezifische Funktionen des Systems mittels einer Sprachsteuerung aktiviert und deaktiviert werden können. Dabei wird ein mit der Spracheingabevorrichtung erfasstes Audiosignal mit einem Sprachanalysemodul verarbeitet, um Sprachbefehle einer Bedienperson zu ermitteln.

Bei der Sprachsteuerung, also der Analyse bzw. der Erkennung einer mittels natürlicher Sprache formulierten Nutzerintention bzw. eines Sprachbefehls kommen bevorzugt Algorithmen der künstlichen Intelligenz, bevorzugt neuronaler Netze zum Einsatz. Diese sind besonders gut geeignet, einen hochdimensionalen Eingangsraum umfassend eine Vielzahl von verschiedenen Sprachsequenzen entsprechend natürlicher Spracheingaben auf einen Zielraum umfassend eine Anzahl definierter Steuerbefehle abzubilden.

Viele medizinische Vorrichtungen müssen für eine Zulassung zudem das Erfordernis der Erstfehlersicherheit bzw. der funktionalen Sicherheit zumindest für ausgewählte Arbeitsschritte bzw. Manöver erfüllen, um die Sicherheit von Patienten und Bedienpersonal zu jeder Zeit während des zunehmend automatisierten Betriebs der medizinischen Vorrichtung zu gewährleisten. Erstfehlersicherheit bedeutet, dass kein einziger erster Fehler dazu führen kann, dass eine Nutzung der medizinischen Vorrichtung während ihrer Lebensdauer unsicher werden kann.

Ein besonders sicherheitskritischer Steuerbefehl ist bspw. auf das Auslösen/Starten einer Röntgenstrahlung bei der Bilddatenerfassung oder der Strahlentherapie gerichtet. Ein anderes Beispiel eines sicherheitskritischen Steuerbefehls betrifft die (autonome) Verstellbewegung einer medizinischen Vorrichtung oder einer ihrer Komponenten, bspw. ein Roboterarm, im Raum. Nicht autorisierte bzw. nicht bestätigte Strahlungsauslösung bzw. Vorrichtungsbewegung kann direkt das Wohl des Patienten bzw. einer Bedienperson gefährden.

Bspw. bei einer medizinischen Prozedur im Rahmen der interventionellen Radiologie, bei der zu verschiedenen Zeitpunkten Röntgenbildaufnahmen mittels Röntgenstrahlung erzeugt werden müssen, kann der die Intervention ausführende Arzt aufgrund seiner Sterilität nicht effektiv bspw. ein manuelles Bedienelement betätigen. Entweder muss der Arbeitsfluss der Prozedur unterbrochen werden, um eine Röntgenbildaufnahme zu starten, oder es muss neben dem Arzt mindestens eine weitere Bedienperson anwesend sein, die die Eingabe des entsprechenden Steuersignals übernimmt.

Um Hardware und/oder Steuerungssoftware einer medizinischen Vorrichtung zur Verarbeitung und Umwandlung von mittels beliebiger Benutzerschnittstelle erfasster Steuerbefehle funktional sicher oder erstfehlersicher auszubilden, ist es heute üblich, eine manuelle Autorisierung eines erkannten Steuerbefehls von einer Bedienperson einzufordern. Ein sogenannter Totmann-Schalter (dead man grip) soll hier als Beispiel dienen. Dieser Schalter/Hebel/Griff muss von einer Bedienperson kontinuierlich betätigt werden, um eine automatische Verstellbewegung an einer medizinischen Vorrichtung zu vollziehen. Die Verstellbewegung stoppt automatisch, wenn die Bedienperson den Totmann-Schalter loslässt. Derart wird eine zufällige oder ungewollte Eingabe eines Steuerbefehls weitgehend vermieden.

Alternativ dazu kann die medizinische Vorrichtung zur Absicherung einer Steuerungssoftware ein zweites, redundantes Softwaresystem auf unabhängiger Hardware, d.h. eigenem Prozessor bzw. eigenem Speicher betrieben. Nur wenn das redundante Softwaresystem den initial erkannten Steuerbefehl plausibilisiert, wird der Steuerbefehl tatsächlich von der medizinischen Vorrichtung ausgeführt, ansonsten wird er verworfen.

Im Bereich der Sprachsteuerung mangelt es jedoch derzeit noch grundlegend an etablierten und verlässlichen Methoden zum Nachweis einer für eine funktionale oder Erstfehlersicherheit erforderlichen Qualität der Spracherkennungsalgorithmen. Dies liegt insbesondere daran, dass die bekannten Methoden zur Sprachverarbeitung noch fehleranfällig und nicht deterministisch sind. Daher mangelt es an universellen Kriterien, denen ein Trainingsdatensatz eines eine Erstfehlersicherheit gewährleistenden (KI-) Spracherkennungsalgorithmus (KI = künstliche Intelligenz) genügen muss oder einem allgemeingültigen Robustheitsmaß eines (KI-) Spracherkennungsalgorithmus zur korrekten Klassifizierung einer bspw. durch Verzerrungen oder Hintergrundgeräusche veränderten Spracheingabe. Dies ist Gegenstand aktueller Forschung. Auch ein an bestehende Sicherheitsnormen angelehntes Vorgehen zur Verifikation eines erkannten Sprachbefehls ist nicht ausreichend sicher bzw. nicht möglich, denn es fehlen auch hier klassisch definierte Anforderungen/Kriterien für KI-Spracherkennungsalgorithmen, um deren Erfüllung nachzuweisen. Dies wäre zwingende Voraussetzung für eine Zulassung bzw. Zertifizierung der (KI-)Spracherkennungsalgorithmen. Daneben würde das Verifizieren eines erkannten Sprachbefehls mittels eines identischen, redundanten Spracherkennungsalgorithmus, der im Zweifel den Erstfehler wiederholt, keine Erstfehlersicherheit gewährleisten.

Infolgedessen kommt eine Sprachsteuerung von medizinischen Vorrichtungen bislang nur außerhalb sicherheitskritischer Anwendungen zum Einsatz.

Aufgabe der vorliegenden Erfindung ist es, dieses Problem zu lösen und Mittel zur Sprachsteuerung einer medizinischen Vorrichtung bereitzustellen, welches eine verbesserte, da verlässlichere Ermittlung von Sprachbefehlen einer Bedienperson aus einem Audiosignal erlaubt. Insbesondere ist es Aufgabe der vorliegenden Erfindung, Mittel bereitzustellen, die eine Erstfehlersicherheit mittels einer Sprachsteuerung gewährleisten.

Diese Aufgabe wird erfindungsgemäß gelöst durch ein Verfahren zur Sprachsteuerung einer medizinischen Vorrichtung, eine entsprechende Sprachsteuervorrichtung, ein medizinisches System umfassend die Sprachsteuervorrichtung, ein Computerprogrammprodukt sowie ein computerlesbares Speichermedium gemäß Hauptanspruch und nebengeordneten Ansprüchen. Vorteilhafte Ausgestaltungen sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind gleichermaßen auf die anderen beanspruchten Gegenstände zu übertragen und umgekehrt. Mit anderen Worten können gegenständliche Ansprüche (die beispielsweise auf eine Sprachsteuervorrichtung gerichtet sind) auch mit Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Merkmale, bspw. Module oder Einheiten, einer der Vorrichtungen ausgebildet.

Die vorliegende Erfindung betrifft in einem ersten Aspekt ein Verfahren zur Sprachsteuerung einer medizinischen Vorrichtung. Das Verfahren ist in Ausführungen als computerimplementiertes Verfahren ausgebildet. Das Verfahren umfasst eine Vielzahl von Schritten.

Ein Schritt ist auf ein Erfassen eines Audiosignals enthaltend eine auf die Steuerung der Vorrichtung gerichtete Spracheingabe einer Bedienperson. Ein Schritt ist auf ein Analysieren des Audiosignals zum Bereitstellen eines ersten Sprachanalyseergebnisses gerichtet. Ein Schritt ist auf ein Erkennen eines ersten Sprachbefehls basierend auf dem ersten Sprachanalyseergebnis gerichtet. Ein Schritt ist auf ein Ermitteln eines Verifikationssignals zur Bestätigung des ersten Sprachbefehls gerichtet.

Das Ermitteln des Verifikationssignals umfasst Teilschritte. Ein Teilschritt betrifft ein Analysieren des Audiosignals zum Bereitstellen eines zweiten Sprachanalyseergebnisses. Ein Teilschritt betrifft ein Erkennen eines zweiten Sprachbefehls der Bedienperson basierend auf dem zweiten Sprachanalyseergebnis. Ein Teilschritt betrifft ein Vergleichen des ersten und des zweiten Sprachbefehls. Das Verifikationssignal bestätigt den ersten Sprachbefehl dann, wenn der erste Sprachbefehl und der zweite Sprachbefehl ein Übereinstimmungskriterium erfüllen.

Ein weiterer Verfahrensschritt ist auf ein Erzeugen eines Steuersignals zur Steuerung der medizinischen Vorrichtung basierend auf dem Verifikationssignal und ggf. dem ersten Sprachbefehl gerichtet. Dieser Schritt wird ausgeführt, sofern der erste Sprachbefehl anhand des Verifikationssignals bestätigt wurde. Dabei ist das Steuersignal geeignet bzw. ausgebildet, die medizinische Vorrichtung entsprechend dem ersten Sprachbefehl zu steuern. Ein weiterer Schritt ist auf eine Eingabe des Steuersignals in die medizinische Vorrichtung gerichtet.

Ein Audiosignal im Sinne der Erfindung kann insbesondere eine Schallinformation enthalten. Das Audiosignal kann ein analoges oder digitales bzw. digitalisiertes Signal sein. Das digitalisierte Signal kann ausgehend von dem analogen Signal z.B. durch einen Analog-Digital-Wandler erzeugt werden. Entsprechend kann der Schritt des Erfassens ein Bereitstellen eines digitalisierten Audiosignals basierend auf dem empfangenen Audiosignal bzw. ein Digitalisieren des empfangenen Audiosignals umfassen. Das Erfassen des Audiosignals kann in Ausführungen ein Registrieren des Audiosignals mittels einer Eingabevorrichtung umfassend einen geeigneten Sensor, bspw. einen akustischen Sensor in Form eines Mikrophons, umfassen, welcher Bestandteil einer Benutzerschnittstelle der medizinischen Vorrichtung sein kann. Das Erfassen des Audiosignals kann ferner ein Bereitstellen des digitalen oder digitalisierten Signals für weitere Analyseschritte umfassen.

Das Audiosignal kann eine Kommunikation von einem Nutzer (im Folgenden auch Bedienperson), bspw. eine auszuführende Anweisung oder Angaben bezüglich der auszuführenden Anweisung, bspw. eine Sprachsteuerinformation, umfassen. Mit anderen Worten kann das Audiosignal eine Spracheingabe der Bedienperson in natürlicher Sprache umfassen. Als natürliche Sprache wird typischerweise eine von Menschen gesprochene Sprache bezeichnet. Natürliche Sprache kann einen Tonfall und/oder eine Sprachmelodie zur Modulation der Kommunikation aufweisen. Natürliche Sprachen können im Gegensatz zu formalen Sprachen strukturelle und lexikalische Uneindeutigkeiten aufweisen.

Ein Analysieren eines Audiosignals ist im Sinne der Erfindung auf das Erschließen bzw. das Erkennen eines Inhaltes des Spracheingabe der Bedienperson gerichtet. Das Analysieren kann das Erkennen einer menschlichen Stimme umfassen. Das Analysieren kann ein Analysieren von Werten der Spracheingabe wie bspw. Frequenz, Amplitude, Modulation oder dergleichen umfassen, die charakteristisch für die menschliche Sprache sind. Das Ergebnis des Analysierens wird zumindest teilweise in Form des ersten Sprachanalyseergebnisses bereitgestellt.

Das erfindungsgemäße Analysieren kann ein Verfahren zur Verarbeitung natürlicher Sprache anwenden. Insbesondere kann gemäß dem ersten Aspekt der Erfindung ein erster Computerlinguistik-Algorithmus auf das Audiosignal bzw. die Spracheingabe angewandt werden. Eine Möglichkeit zur Verarbeitung der in natürlicher Sprache formulierten Spracheingabe zur Bereitstellung des ersten Sprachanalyseergebnisses ist ein Umwandeln der in natürlicher Sprache formulierten Spracheingabe in Text, also in strukturierte Sprache, mittels eines Sprachezu-Text (Software-)Moduls. Anschließend kann eine weitere Analyse zur Bereitstellung des ersten Sprachanalyseergebnisses, beispielsweise mittels latenter semantischer Analyse (engl. latent semantic indexing, kurz LSI), dem Text eine Bedeutung beimessen. Insbesondere wird hierbei das Audiosignal als Ganzes analysiert. Es kann vorgesehen sein, dabei einzelne Worte oder Wortgruppen und/oder eine Syntax zu erkennen. Die Beziehung/Stellung von Worten oder Wortgruppen zu vorher oder später in dem Audiosignal erscheinenden Worten oder Wortgruppen kann ebenfalls berücksichtigt werden. Die Analyse des Audiosignals kann insbesondere eine grammatikalische Analyse bspw. unter Anwendung eines Sprach- oder Grammatikmodells umfassen. Zusätzlich oder alternativ kann eine Sprachmelodie der Bedienperson ausgewertet werden. Derart realisiert die Erfindung ein Verstehen der in der Spracheingabe enthaltenen natürlichen Sprache (engl.: natural language understanding, NLU).

Der Schritt des Erkennens eines ersten Sprachbefehls basierend auf dem ersten Sprachanalyseergebnis ist darauf gerichtet, anhand des Sprachanalyseergebnisses der in der Spracheingabe erkannten Bedeutung des umfassten Textes einen eindeutigen Sprachbefehl zuzuordnen bzw. mit einem vordefinierten Satz möglicher Sprachbefehle in Verbindung zu bringen. Dabei kann der Spracheingabe im Schritt des Erkennens des ersten Sprachbefehls basierend auf dem Sprachanalyseergebnis eine beliebige Vielzahl von verschiedenen Sprachbefehlen zugeordnet werden. Jeder Sprachbefehl ist dabei repräsentativ für eine auszuführende Anweisung zur Ausführung eines Arbeitsschritts, einer definierten Aktion, Operation oder Bewegung, die basierend auf dem Sprachbefehl automatisch von der medizinischen Vorrichtung ausgeführt wird. Das Erkennen des ersten Sprachbefehls kann eine Klassifizierung der Spracheingabe anhand des Sprachanalyseergebnisses in eine Befehlsklasse eines vordefinierten Satzes verschiedener Befehlsklassen umfassen. Der vordefinierte Satz möglicher Befehlsklassen kann z.B. in Form einer Befehlsbibliothek vorliegen. Insbesondere können mehrere voneinander abweichende Spracheingaben bzw. ihre individuellen Sprachanalyseergebnisse ihrem Bedeutungsgehalt nach derselben Befehlsklasse und damit demselben Sprachbefehl zugeordnet werden.

Derart wird der Dimensionalität der natürlichen Sprache Rechnung getragen, bei der unter anderem mittels unterschiedlicher Wortwahl oder Sprachmelodie einund dieselbe Nutzerintention ausgedrückt werden kann.

Die Befehlsbibliothek kann auch eine Befehlsklasse für nicht erkannte Sprachbefehle umfassen, die immer dann zugeordnet wird, wenn das Audiosignal akustische Merkmale aufweist, die unspezifisch für einen konkreten Sprachbefehl sind bzw. sich nicht einer anderen Befehlsklasse zuordnen lässt.

Auch der Schritt des Erkennens des ersten Sprachbefehls kann durch Anwenden des ersten Computerlinguistik-Algorithmus ausgeführt werden. Der Schritt des Erkennens des ersten Sprachbefehls folgt dabei bevorzugt dem Erstellen eines Transkripts (strukturierte Sprache) des Sprachsignals bzw. dem auf dem Transkript basierenden Erstellen eines semantischen Analyseergebnisses. Beides kann in dem ersten Sprachanalyseergebnis umfasst sein.

Die Vielzahl der Befehlsklassen ist in Implementierungen zumindest teilweise spezifisch für die medizinische Vorrichtung und richtet sich insbesondere an der bereitgestellten Funktionalität der medizinischen Vorrichtung aus. Die Vielzahl der Befehlsklassen ist in Implementierungen anpassbar ausgebildet und kann insbesondere über die Auswahl bzw. die Konfiguration bzw. das Training des bei der Analyse eingesetzten Computer-linguistik-Algorithmus angepasst werden. Bspw. können weitere Befehlsklassen hinzugefügt werden.

In einem weiteren Schritt wird ein Verifikationssignal zur Bestätigung des ersten Sprachbefehls ermittelt. In diesem Schritt wird überprüft, ob der erste, identifizierte Sprachbefehl tatsächlich der von der Bedienperson gewünschten Nutzerintention bzw. dem gewünschten Kommando entspricht. Das Verifikationssignal gibt dabei ein Maß für die Übereinstimmung zwischen gewünschter Benutzerintention und dem ersten Sprachbefehl an. In Implementierungen kann das Verifikationssignal eine Verifikationsinformation umfassen. Die Verifikationsinformation kann wenigstens zwei, bspw. ‚1' für vollständige Übereinstimmung und ‚0' für keine Übereinstimmung, in Ausführungen auch mehrere verschiedene diskrete Werte, bspw. zwischen ‚1' und ‚0' annehmen. Einige der diskreten Werte können verschiedenen Übereinstimmungsniveaus entsprechen, die jeweils einer anteiligen, nicht vollständigen Übereinstimmung entsprechen können. Auch diese Werte können in Ausführungen der Erfindung einer auseichenden Übereinstimmung zwischen Sprachbefehl und gewünschter Nutzerintention entsprechen.

Der Schritt des Ermittelns des Verifikationssignals wird erfindungsgemäß auf folgende Weise ausgeführt:

Das Audiosignal wird analysiert, um ein zweites Sprachanalyseergebnis bereitzustellen.

Auch in dieser Analyse wird der Inhalt der Spracheingabe der Bedienperson erkannt bzw. erschlossen. Auch dieses Analysieren kann das Erkennen einer menschlichen Stimme umfassen. Das Analysieren kann ein Analysieren von Werten der Spracheingabe wie bspw. Frequenz, Amplitude, Modulation oder dergleichen umfassen, die charakteristisch für die menschliche Sprache sind. Das Ergebnis des Analysierens wird zumindest teilweise in Form des zweiten Sprachanalyseergebnisses bereitgestellt.

Auch hier kann ein Verfahren zur Verarbeitung natürlicher Sprache zum Einsatz kommen. Insbesondere kann hierbei ein zweiter Computerlinguistik-Algorithmus auf das Audiosignal bzw. die Spracheingabe angewandt werden. Insbesondere kann die Analyse auch eine Verarbeitung der in natürlicher Sprache formulierten Spracheingabe zur Bereitstellung des zweiten Sprachanalyseergebnisses ein Umwandeln der Spracheingabe in Text, also in strukturierte Sprache, mittels eines Sprachezu-Text (Software-)Moduls umfassen. Anschließend kann eine weitere Analyse zur Bereitstellung des zweiten Sprachanalyseergebnisses, beispielsweise mittels latenter semantischer Analyse (engl. latent semantic indexing, kurz LSI), dem Text eine Bedeutung beimessen. Auch hier wird das Audiosignal bevorzugt als Ganzes analysiert. Dabei können einzelne Worte oder Wortgruppen und/oder eine Syntax erkannt werden. Die Beziehung/Stellung von Worten oder Wortgruppen zu vorher oder später in dem Audiosignal erscheinenden Worten oder Wortgruppen kann ebenfalls berücksichtigt werden. Die Analyse des Audiosignals kann insbesondere eine grammatikalische Analyse bspw. unter Anwendung eines Sprach- oder Grammatikmodells umfassen. Zusätzlich oder alternativ kann eine Sprachmelodie der Bedienperson ausgewertet werden.

Der Schritt des Erkennens des zweiten Sprachbefehls basierend auf dem zweiten Sprachanalyseergebnis ist ebenfalls darauf gerichtet, anhand des zweiten Sprachanalyseergebnisses der in der Spracheingabe erkannten Bedeutung des umfassten Textes einen eindeutigen Sprachbefehl zuzuordnen bzw. mit einem vordefinierten Satz möglicher Sprachbefehle in Verbindung zu bringen. Dabei kann der Spracheingabe im Schritt des Erkennens des zweiten Sprachbefehls basierend auf dem zweiten Sprachanalyseergebnis eine definierte Menge verschiedener Sprachbefehlen zugeordnet werden. Jeder Sprachbefehl ist dabei wieder repräsentativ für eine konkrete Anweisung zur Ausführung eines Arbeitsschritts, einer definierten Aktion, Operation oder Bewegung, die basierend auf dem Sprachbefehl automatisch von der medizinischen Vorrichtung ausgeführt wird.

Auch der Schritt des Erkennens des zweiten Sprachbefehls kann durch Anwenden des zweiten Computerlinguistik-Algorithmus ausgeführt werden. Der Schritt des Erkennens des zweiten Sprachbefehls folgt dabei bevorzugt ebenfalls dem Erstellen eines Transkripts (strukturierte Sprache) des Sprachsignals bzw. dem auf dem Transkript basierenden Erstellen eines semantischen Analyseergebnisses. Beides kann in dem ersten Sprachanalyseergebnis umfasst sein.

Erfindungsgemäß wird das Audiosignal ein zweites Mal, insbesondere unabhängig vom ersten Analyseschritt, wie weiter unten näher beschrieben wird, analysiert, und ein zweites Sprachanalyseergebnis generiert, um darauf basierend einen zweiten Sprachbefehl bereitzustellen, der dann mit dem ersten Sprachbefehl verglichen wird. Das erste und das zweite Sprachanalyseergebnis können sich in Ausführungen unterscheiden, obwohl beide Sprachanalyseergebnisse basierend auf demselben Audiosignal erzeugt werden. Auch der erste und der zweite Sprachbefehl können sich voneinander unterscheiden. Ursache dafür ist die verschiedenartige Ausbildung der jeweiligen Analyseschritte zur Identifikation der Sprachbefehle, wie weiter unten noch genauer beschrieben wird.

Es folgt ein Vergleichen des ersten und des zweiten Sprachbefehls, wobei das Verifikationssignal den ersten Sprachbefehl bestätigt, wenn der erste Sprachbefehl und der zweite Sprachbefehl ein Übereinstimmungskriterium erfüllen. Das Verifikationssignal basiert also erfindungsgemäß auf einem Vergleich zwischen dem ersten und dem zweiten Sprachbefehl. Wird eine Übereinstimmung zwischen dem ersten und dem zweiten Sprachbefehl ermittelt, wird eine den ersten Sprachbefehl bestätigende Verifikationsinformation für das Verifikationssignal erzeugt. Das Ermitteln einer Übereinstimmung zwischen erstem und zweitem Sprachbefehl entspricht dabei einem erfindungsgemäßen Prüfschritt bzw. Bestätigungszyklus für den ersten Sprachbefehl.

Ein Übereinstimmungskriterium kann als ein Schwellwert für die Gleichheit bzw. Ähnlichkeit des ersten und des zweiten Sprachbefehls ausgebildet sein. Der Schwellwert kann einen Wert von 100% indikativ für eine Identität zwischen erstem und zweiten Sprachbefehl annehmen. Der Schwellwert kann aber auch kleiner als 100% sein. Der Schwellwert für das einzuhaltende Übereinstimmungskriterium kann bspw. von einer Sicherheitsklasse bzw. von einer Sicherheitsanforderung abhängen, welcher der erste und/oder der zweite Sprachbefehl zugeordnet ist bzw. welche für den jeweiligen Sprachbefehl entsprechend des auszuführenden Arbeitsschritts vorgesehen ist. Entsprechend können erfindungsgemäß insbesondere von dem zweiten Sprachbefehl abhängige Schwellwerte für die Menge der möglichen zweiten Sprachbefehle hinterlegt sein, wobei die Schwellwerte ein jeweiliges Sicherheitslevel entsprechend einer Sicherheitsklasse eines Steuerbefehls repräsentieren können.

Eine Sicherheitsklasse eines Steuerbefehls steht jeweils für ein Sicherheitsmaß bzw. Sicherheitslevel, welches bei Ausführung des/der dem jeweiligen Steuerbefehl entsprechenden Arbeitsschritt, Operation bzw. Manöver durch die medizinische Vorrichtung eingehalten werden muss. Ein Steuerbefehl kann auch Sprachbefehle umfassen, die ebenfalls den vorgegebenen Sicherheitsanforderungen gerecht werden müssen. Das jeweilige Sicherheitsmaß kann dabei ein mittels Normierung festgelegtes Sicherheitsmaß sein. Erfindungsgemäß kann jeder Sprachbefehl bzw. jede Befehlsklasse einer Sicherheitsklasse zugeordnet sein. Mit anderen Worten existiert erfindungsgemäß eine vorbestimmte Menge verschiedener Sicherheitsklassen, jeweils repräsentierend ein unterschiedliches Sicherheitsmaß. Jeder mögliche Sprachbefehl, der als erster und/oder zweiter Sprachbefehl identifiziert werden kann, kann demnach seinen Sicherheitsanforderungen entsprechend einer der Sicherheitsklassen zugeordnet sein. Diese Zuordnungsvorschrift kann regelbasiert oder mittels Lookup-Tabelle vorab festgelegt sein. Mehrere Sprachbefehle können einer Sicherheitsklasse zugewiesen sein. Insbesondere kann mindestens eine Sicherheitsklasse vorgesehen sein, die sicherheitskritische, insbesondere erstfehlersicher abzubildende Sprachsteuerbefehle umfasst. Es können aber auch mehrere Sicherheitsklassen für sicherheitskritische Sprachbefehle entsprechend verschiedener sicherheitsrelevanter Sicherheitsstufen vorgesehen sein. Insbesondere kann auch wenigstens eine Sicherheitsklasse für nicht sicherheitskritische Sprachbefehle bzw. allgemeine Spracheingaben vorgesehen sein. Die Klassifizierung der möglichen Sprachbefehle kann vorab in Abhängigkeit der medizinischen Vorrichtung, ihrer Funktionalitäten und/oder entsprechender Sicherheitsvorgaben für die per Sprachbefehl zu steuernden Arbeitsschritte festgelegt sein.

Erfindungsgemäß wird nach Bestätigung des ersten Sprachbefehls mittels des Verifikationssignals dasselbe zur Weiterverarbeitung bspw. einer Steuereinheit einer erfindungsgemäßen Sprachsteuervorrichtung bereitgestellt. Insofern kann der Schritt des Erzeugens eines Steuersignals basierend auf dem Verifikationssignal und ggf. dem ersten Sprachbefehl ein Bereitstellen derselben umfassen. Das Verifikationssignal zeigt an, ob das für den ersten Sprachbefehl geforderte Gleichheits- oder Ähnlichkeitsmaß erreicht ist oder nicht. Bei entsprechender Bestätigung des ersten Sprachbefehls mittels des Verifikationssignals, wird in einem folgenden Schritt ein Steuersignal zur Steuerung der medizinischen Vorrichtung basierend auf dem ersten Sprachbefehl und dem Verifikationssignal erzeugt und in die medizinische Vorrichtung eingegeben.

Die auf das Ableiten des zweiten Sprachbefehls gerichteten Schritte sowie der Vergleich des ersten und des zweiten Sprachbefehls können in Ausführungen der Erfindung einen P-Pfad ausbilden, der vorteilhaft ohne eine weitere Nutzerinteraktion auskommt. Die auf das Ableiten des zweiten Sprachbefehls aus ein und demselben Audiosignal gerichteten Schritte sowie der Vergleich des ersten und des zweiten Sprachbefehls können zumindest teilweise zeitlich parallel zum Ableiten des ersten Sprachbefehls erfolgen. Bspw. kann in Implementierungen das erfasste Audiosignal zeitgleich dem ersten und dem zweiten Analyseschritt zugeführt werden. Zudem kann zeitgleich der erste und der zweite Sprachbefehl identifiziert werden, da der erste und der zweite Computerlinguistik-Algorithmus voneinander unabhängig und insbesondere in verschiedenen Software-/Hardware-Umgebungen implementiert sind. Alternativ können diese Schritte zum Ermitteln des zweiten Sprachbefehls zeitlich nach dem Ableiten des ersten Sprachbefehls durchgeführt werden.

Mit dem Schritt des Ermittelns des Verifikationssignals wendet die vorliegende Erfindung vorteilhaft eine Überwachung bzw. Plausibilisierung eines Sprachbefehls, also eines mittels Methoden der maschinellen Sprachanalyse gewonnenen Steuerbefehls, an. Mit anderen Worten ermöglicht die vorliegende Erfindung mit diesen Schritten, einen Prüf- (P-protect) Pfad für Sprachbefehle entsprechend eines erstfehlersicheren Systems zu implementieren. Wird der erste Sprachbefehl durch den Plausibilisierungsschritt bestätigt (Verifikationssignal zeigt eine Übereinstimmung), wird der erste Sprachbefehl weiterverarbeitet und durch die medizinische Vorrichtung ausgeführt. Wird der erste Sprachbefehl durch den Plausibilisierungsschritt nicht bestätigt (Verifikationssignal zeigt eine Abweichung), wird der Prozess abgebrochen und der erste Sprachbefehl wird verworfen und nicht ausgeführt.

Die Erfindung ermöglicht mit der Ermittlung des Verifikationssignals vorteilhaft, dass die Ausführung von mittels maschineller Spracherkennung falsch erkannten bzw. falsch interpretierten Nutzereingaben verhindert wird. Insbesondere wird derart die sicherheitskritische bzw. erstfehlersichere Ausführung von Steuerbefehlen ermöglicht. Der erste erkannte Sprachbefehl wird nur dann ausgeführt, wenn dieser mittels des Plausibilisierungsschritts bestätigt wurde.

Das Ermitteln des Verifikationssignals wird deshalb in Ausführungen der Erfindung vorteilhaft in einem getrennten, unabhängigen Hard- und/oder Softwaresystem implementiert. Insbesondere die Ermittlung des Verifikationssignals erfolgt also unabhängig von den übrigen Prozessschritten. Algorithmen zur Ermittlung des Verifikationssignals unterscheiden sich insbesondere von den zuvor im Verfahren eingesetzten Computerlinguistik-Algorithmen. Der Schritt des Ermittelns des Verifikationssignals wird in Ausführungen der Erfindung in einer anderen realen oder virtuellen Recheneinheit ausgeführt als die vorherigen Prozessschritte. Dadurch kann erfindungsgemäß ein erstfehlersicheres System umfassend einen Steuer-Pfad (C-Pfad, C-control) und einen Prüf-Pfad (P-Pfad, P-protect) eingerichtet werden, wobei der Schritt des Ermittelns des Verifikationssignals innerhalb des abgesicherten P-Pfades erfolgt. Insbesondere die Schritte des Erfassens eines Audiosignals enthaltend eine auf die Steuerung der Vorrichtung gerichtete Spracheingabe einer Bedienperson, des ersten Analysierens des Audiosignals zum Bereitstellen eines ersten Sprachanalyseergebnisses und des Erkennens eines ersten Sprachbefehls basierend auf dem ersten Sprachanalyseergebnis bilden einen wesentlichen Bestandteil des C-Pfades.

Wie eingangs erwähnt, umfasst in weiteren bevorzugten Implementierungen der Erfindung umfasst das Analysieren zum Bereitstellen des ersten Sprachanalyseergebnisses ein Anwenden eines ersten Computerlinguistik-Algorithmus. Dieser kann bevorzugt eine erste trainierte Funktion umfassen, die auf das Audiosignal angewandt wird. Das Analysieren zum Bereitstellen eines zweiten Sprachanalyseergebnisses umfasst weiter ein Anwenden eines zweiten Computerlinguistik-Algorithmus umfassend eine zweite trainierte Funktion, die auf das Audiosignal angewendet wird. Dabei unterscheiden sich die erste trainierte Funktion und die zweite trainierte Funktion voneinander.

In bevorzugter Weiterbildung ist nämlich die zweite trainierte Funktion ausgebildet, nur sicherheitskritische Sprachbefehle zu erkennen.

Ein sicherheitskritischer Sprachbefehl ist dabei repräsentativ für einen definierte Arbeitsschritt, eine Aktion, Operation oder Bewegung, die basierend auf dem Sprachbefehl automatisch von der medizinischen Vorrichtung ausgeführt werden soll und die definierten Sicherheitsanforderungen, insbesondere genormten Sicherheitsanforderungen gerecht werden muss. Sicherheitskritische Sprachbefehle umfassen insbesondere auch erstfehlersichere Sprachbefehle. Erstfehlersichere Sprachbefehle sind als diejenigen sicherheitskritischen Sprachbefehle mit den höchsten Sicherheitsanforderungen zu verstehen. Sicherheitskritische Sprachbefehle können in Implementierungen genau einer oder eine Vielzahl von Sicherheitsklassen zugeordnet sein, wobei im letzten Fall auch die sicherheitskritischen Sprachbefehle als Untermenge aller möglichen Sprachbefehle verschiedene Sicherheitsstufen ausweisen können. Sicherheitskritische Sprachbefehle, die der zweite Computerlinguistik-Algorithmus erkennen soll, weisen typischerweise eine charakteristische Merkmalskombination, also eine charakteristische akustische Signalverläufe, Frequenzmuster, Amplitude, Modulation oder dergleichen auf. Sie können zudem eine charakteristische und leicht zu diskriminierende Buchstaben- und oder Wortfolge aufweisen. Sicherheitskritische, insbesondere erstfehlersichere Sprachbefehle können bspw. durch eine Mindest-Silbenanzahl gekennzeichnet sein, die bspw. bei drei oder mehr liegt. Oder sie weisen eineindeutige, also kaum verwechselbare phonetische Merkmale auf, sodass eine Ähnlichkeit zu anderen Sprachbefehlen oder allgemein anderen Spracheingaben und eine damit einhergehende Verwechslungsgefahr von vornherein minimiert ist. Die genannten Eigenschaften bzw. Merkmale des Audiosignals können in den erfindungsgemäßen Schritten des Analysierens des Audiosignals erfasst und als Sprachanalyseergebnis bereitgestellt werden.

Entsprechend ist in bevorzugter Implementierung der Erfindung die zweite trainierte Funktion ausgebildet, anhand der zweiten Tokenisierungsinformation und/oder der zweiten semantischen Information, allgemeiner gesagt anhand des zweiten Sprachanalyseergebnisses, strukturell und/oder lexikalisch eindeutige Sprachbefehle als sicherheitskritische Sprachbefehle zu identifizieren.

Es werden erfindungsgemäß zumindest durch den zweiten Computerlinguistik-Algorithmus strukturelle Eigenschaften und/oder lexikalische Eigenschaften des Audiosignals ermittelt. Erfindungsgemäß sind also charakteristische, eindeutige strukturelle und/oder lexikalische Eigenschaften mit sicherheitskritischen Sprachbefehlen verknüpft. Erfindungsgemäß werden folglich sicherheitskritischen Sprachbefehlen vorab genau diese eindeutigen Eigenschaften zugeordnet, um sicherheitskritische Sprachbefehle leicht und verlässlich als solche identifizieren zu können. Anders ausgedrückt, können erfindungsgemäß basierend auf charakteristischen, strukturellen und lexikalischen Eigenschaften/Merkmalen Audiosignale entsprechend verlässlich identifizierbarer sicherheitskritischer Sprachbefehle vorgegeben sein.

In diesem Sinne ist in besonders bevorzugter Ausführung die zweite trainierte Funktion ausgebildet, einen Sprachbefehl mit mindestens drei, bevorzugt vier Silben als sicherheitskritischen Sprachbefehl zu identifizieren. Hierbei geht die Erfindung davon aus, dass die Verwechslungsgefahr eines Sprachbefehls mit der Anzahl der umfassten Silben sinkt. Besonders bevorzugt werden also sicherheitskritische Arbeitsschritte der Vorrichtung mit Sprachbefehlen umfassend mindestens drei Silben belegt, besonders bevorzugt mit Sprachbefehlen umfassend fünf bis sieben Silben.

Der erste Computerlinguistik-Algorithmus umfasst also eine erste trainierte Funktion, die zu dessen Analyse auf das Audiosignal angewandt wird. Die trainierte Funktion ist als ein trainierter Algorithmus des maschinellen Lernens zu verstehen. Bevorzugt umfassen auch das Erkennen eines ersten Sprachbefehls basierend auf dem ersten Sprachanalyseergebnis sowie das Zuordnen des ersten Sprachbefehls zu einer Sicherheitsklasse ein Anwenden des ersten Computerlinguistik-Algorithmus.

Bevorzugt umfasst die trainierte Funktion bzw. der trainierte Algorithmus des maschinellen Lernens ein neuronales Netz, bevorzugt ein faltendes neuronales Netz. Ein neuronales Netz ist i.W. wie ein biologisches neuronales Netz aufgebaut, bspw. wie das menschliche Gehirn. Bevorzugt umfasst ein künstliches, neuronales Netz eine Eingabeschicht und eine Ausgabeschicht. Dazwischen kann es eine Vielzahl von Zwischenschichten umfassen. Jede der Schichten umfasst einen, bevorzugt eine Vielzahl von Knoten. Jeder Knoten wird dabei als biologische Recheneinheit bzw. Schaltstelle, bspw. als Neuron angesehen. Anders ausgedrückt, entspricht ein einzelner Knoten einer bestimmten auf die Eingabedaten angewandten Rechenoperation. Knoten einer Schicht können untereinander über entsprechende Ränder oder Verbindungen miteinander und/oder zu Knoten anderer Schichten verbunden sein, speziell über gerichtete Verbindungen. Diese Ränder bzw. Verbindungen definieren den Datenfluss des Netzes. In bevorzugten Ausführungen ist ein Rand/eine Verbindung mit einem Parameter ausgestattet, wobei der Parameter auch als "Gewicht" bezeichnet wird. Dieser Parameter reguliert den Einfluss bzw. das Gewicht der Ausgabedaten eines ersten Knotens für die Eingabe eines zweiten Knotens, der über die Verbindung mit dem ersten Knoten in Kontakt steht.

Gemäß Ausführungen der Erfindung ist das neuronale Netz ein trainiertes Netz. Das Training des neuronalen Netzes wird bevorzugt im Sinne eines 'überwachten Lernens' basierend auf Trainingsdaten eines Trainingsdatensatzes, nämlich bekannten Paaren von Eingabe- und Ausgabedaten durchgeführt. Dabei werden die bekannten Eingabedaten als Eingabedaten an das neuronale Netz übergeben und die Ausgabedaten des neuronalen Netzes werden mit den bekannten Ausgabedaten des Trainingsdatensatzes verglichen. Das künstliche neuronale Netz lernt nun selbständig und passt die Gewichte der einzelnen Knoten bzw. Verbindungen derart und solange an, bis die Ausgabedaten der Ausgabeschicht des neuronalen Netzes ausreichend ähnlich zu den bekannten Ausgabedaten des Trainingsdatensatzes sind. In diesem Zusammenhang spricht man bei faltenden neuronalen Netzen auch von 'deep learning'. Die Termini 'neuronales Netz' und 'künstliches neuronales Netz' können als Synonyme verstanden werden.

Erfindungsgemäß wird in Ausführungen das faltende neuronale Netz, also die trainierte Funktion des ersten Computerlinguistik-Algorithmus in einer Trainingsphase darauf trainiert, das erfasste Audiosignal zu analysieren und einen ersten Sprachbefehl bzw. eine Befehlsklasse entsprechend dem ersten Sprachbefehl daraus zu erkennen. Der erste Sprachbefehl und/oder die Befehlsklasse entspricht dann den Ausgabedaten der trainierten Funktion.

Sprachbefehle entsprechend der Gruppe von Steuerbefehlen, die der erste Computerlinguistik-Algorithmus erkennen soll, können eine Vielzahl von akustischen, strukturellen und/oder lexikalischen Merkmalskombinationen aufweisen, also eine Vielzahl von verschiedenen Kombinationen aus Frequenzmuster, Amplituden, Modulationen, Worten, Wortfolgen, Silben oder dergleichen. Entsprechend lernt die trainierte Funktion in der Trainingsphase, anhand einer aus dem Audiosignal extrahierten Merkmalskombination im Sinne des ersten Sprachanalyseergebnisses einen der möglichen Sprachbefehle zuzuordnen bzw. das Audiosignal entsprechend einer der Befehlsklassen zu klassifizieren. Die Trainingsphase kann auch eine manuelle Zuordnung von Trainings-Eingabedaten in Form von Spracheingaben zu einzelnen Sprachbefehlen bzw. Befehlsklassen umfassen. Die erste trainierte Funktion ist darauf trainiert, einen möglichst breiten und vielseitigen Satz von Audiosignalen umfassend menschliche Sprache als einen Sprachbefehl auf einer vorab definierten, insbesondere großen Menge von Befehlsklassen zu erkennen.

Eine erste Gruppe der neuronalen Netz-Schichten kann auf das Extrahieren bzw. das Ermitteln der akustischen Merkmale des Audiosignals gerichtet sein, also das Bereitstellen des ersten Sprachanalyseergebnisses umfassend eine für das Audiosignal spezifische Kombination akustischer Merkmale. Das erste Sprachanalyseergebnis kann in Form eines akustischen Merkmalsvektors bereitgestellt werden. Insofern dient ein das Audiosignal bevorzugt vollständig umfassender Sprachdatenstrom als Eingabedaten für das neuronale Netz. Das erste Sprachanalyseergebnis kann als Eingabedaten für eine zweite Gruppe neuronaler Netz-Schichten dienen, die auch als 'Classifier' bezeichnet werden. Die zweite Gruppe neuronaler Netz-Schichten dient dazu, dem extrahierten Merkmalsvektor wenigstens einen Sprachbefehl bzw. eine Befehlsklasse zuzuordnen. Die Menge von Befehlsklassen kann dabei insbesondere auch eine Befehlsklasse für nicht erkannte Sprachbefehle umfassen. Das neuronale Netz kann entsprechend darauf trainiert sein, das Audiosignal dieser Klasse zuzuordnen, wenn kein Sprachbefehl anhand der Merkmale eindeutig erkannt werden kann.

Die Analyseschritte bzw. die Funktionen können auch von mehreren, insbesondere zwei oder drei eigenständigen neuronalen Netzen ausgeführt werden. Der erste Computerlinguistik-Algorithmus kann folglich ein oder mehrere neuronale Netze umfassen. Zum Beispiel kann die Merkmalsextraktion mit einem ersten neuronalen Netz, und die Klassifizierung mit einem zweiten neuronalen Netz ausgeführt werden.

Das Klassifizieren eines Audiosignals in eine Befehlsklasse basierend auf dem ersten Sprachanalyseergebnis kann auf einem Vergleich des extrahierten Merkmalsvektors des Audiosignals mit in der Befehlsbibliothek hinterlegten, für jede Befehlsklasse spezifischen Merkmalsvektoren basieren. Für eine Befehlsklasse können ein oder mehrere Merkmalsvektoren hinterlegt sein, um der Vieldimensionalität der menschlichen Sprache Rechnung zu tragen und basierend auf einer Vielzahl von verschiedenartigen Spracheingaben einen konkreten Sprachbefehl identifizieren zu können.

Der Vergleich der Merkmalsvektoren kann einen individuellen Abgleich einzelner, bevorzugt aller von Merkmalsvektor umfassten Merkmale umfassen. Alternativ oder zusätzlich kann der Vergleich auf einem aus dem Merkmalsvektor abgeleiteten Merkmalsparameter basieren, welche Einzelmerkmale berücksichtigt. Das sich bei dem Vergleich ergebende Übereinstimmungsmaß für die Merkmalsvektoren oder Merkmalsparameter gibt an, welcher Sprachbefehl bzw. welche Befehlsklasse zugeordnet wird. Es wird, die Befehlsklasse zugeordnet, die die größte Ähnlichkeit bzw. eine Ähnlichkeit oberhalb eines festgelegten Schwellwertes hat.

Der Schwellwert für das festgelegte Ähnlichkeitsmaß kann automatisch oder durch eine Bedienperson voreingestellt werden. Es kann ferner von der für das Audiosignal erkannten spezifischen Merkmalskombination abhängen. Der Schwellwert kann eine Vielzahl von Einzelschwellwerten für individuelle Merkmale des Merkmalsvektors oder einen universellen Schwellwert berücksichtigend die Vielzahl der im Merkmalsvektor umfassten Einzelmerkmale darstellen.

Das weitere Analysieren des Audiosignals kann nun ein Anwenden eines zweiten Computerlinguistik-Algorithmus umfassend eine zweite trainierte Funktion auf das Audiosignal umfassen. Bevorzugt umfasst auch das Erkennen des zweiten Sprachbefehls basierend auf dem zweiten Sprachanalyseergebnis ein Anwenden des zweiten Computerlinguistik-Algorithmus. Auch das Vergleichen von erstem und zweitem Sprachbefehl kann mittels der zweiten trainierten Funktion ausgeführt werden. Erste und zweite trainierte Funktion sind erfindungsgemäße verschiedene trainierte Funktionen.

Die zweite trainierte Funktion ist, wie eingangs erwähnt, in Implementierungen ausgebildet, nur sicherheitskritische Sprachbefehle in dem Audiosignal zu identifizieren. In diesem Sinne ist die zweite trainierte Funktion bzw. der zweite Computerlinguistik-Algorithmus spezifisch für sicherheitskritische Sprachbefehle umfassend auch erstfehlersichere Sprachbefehle.

Der Schritt des Erkennens des zweiten Sprachbefehls basierend auf dem zweiten Sprachanalyseergebnis ist insbesondere darauf gerichtet, das zweite Sprachanalyseergebnis mit einem vordefinierten Satz sicherheitskritischer, insbesondere erstfehlersicherer Sprachbefehle in Verbindung zu bringen. Das Erkennen des zweiten Sprachbefehls umfasst also das Erkennen eines sicherheitskritischen, insbesondere eines erstfehlersicheren Sprachbefehls.

Auch die zweite trainierte Funktion bzw. der zweite trainierte Algorithmus des maschinellen Lernens umfasst ein neuronales Netz, welches grundsätzlich wie mit Bezug zur ersten trainierten Funktion beschrieben, ausgebildet sein kann. Gemäß Ausführungen der Erfindung ist auch das zweite neuronale Netz ein trainiertes Netz. Das Training des neuronalen Netzes wird bevorzugt im Sinne eines überwachten Lernens' basierend auf Trainingsdaten eines Trainingsdatensatzes, nämlich bekannten Paaren von Eingabe- und Ausgabedaten durchgeführt, wie oben beschrieben. Erfindungsgemäß wird also in Ausführungen das zweite neuronale Netz, also die zweite trainierte Funktion des zweiten Computerlinguistik-Algorithmus in einer Trainingsphase darauf trainiert, das erfasste Audiosignal zu analysieren und einen zweiten Sprachbefehl als sicherheitskritischen, insbesondere erstfehlersicheren Sprachbefehle aus einer Menge der sicherheitskritischen Sprachsteuerbefehle zu identifizieren. Der zweite Sprachbefehl kann in Ausführungen den Ausgabedaten der zweiten trainierten Funktion entsprechen. In anderen Ausführungen übernimmt die zweite trainierte Funktion auch den Schritt des Vergleichens des ersten und des zweiten Sprachbefehls.

Sicherheitskritische Sprachbefehle, die der zweite Computer-linguistik-Algorithmus erkennen soll, weisen eine wie eingangs beschriebene charakteristische Merkmalskombination auf. Diese unterscheidet sich insbesondere von der Merkmalskombination derjenigen Sprachbefehle, die der erste Computerlinguistik-Algorithmus erkennen soll. Sicherheitskritische, insbesondere erstfehlersichere Sprachbefehle weisen eindeutige, kaum verwechselbare phonetische, lexikalische bzw. strukturelle Merkmale auf, die kaum mit Merkmalen anderer Sprachbefehlen verwechselt werden können.

Entsprechend lernt auch die zweite trainierte Funktion in der Trainingsphase, anhand einer aus dem Audiosignal extrahierten Merkmalskombination im Sinne des zweiten Sprachanalyseergebnisses einen der sicherheitskritischen Sprachbefehle zuzuordnen. Die Trainingsphase kann auch eine manuelle Zuordnung von Trainings-Eingabedaten in Form von Spracheingaben zu einzelnen Sprachbefehlen umfassen.

Wie bei der ersten trainierten Funktion kann auch bei der zweiten trainierten Funktion eine erste Gruppe der neuronalen Netz-Schichten auf das Extrahieren bzw. das Ermitteln der akustischen Merkmale eines Audiosignals und das Bereitstellen des zweiten Sprachanalyseergebnisses gerichtet sein. Es wird auf die vorherige Beschreibung verwiesen, die hier übertragen werden kann. Das zweite Sprachanalyseergebnis kann ebenfalls als Eingabe-Datensatz für eine zweite Gruppe neuronaler Netz-Schichten, dem ,Classifier', dienen, die dem zweiten Sprachanalyseergebnis einen der sicherheitskritischen Sprachbefehle zuordnet. Die zweite trainierte Funktion kann vorteilhaft ausgebildet sein, dem zweiten Sprachbefehl eine Fehler-Ausgabe zuzuordnen, wenn kein sicherheitskritischen Sprachbefehl erkannt wurde. Erkennt die zweite trainierte Funktion keinen sicherheitskritischen Sprachbefehl, wird der erste Sprachbefehl im Sinne einer Sicherheit von Mensch und Gerät immer verworfen.

Auch hier können die einzelnen Funktionen alternativ von mehreren, insbesondere zwei eigenständigen neuronalen Netzen ausgeführt werden.

Das Klassifizieren des Audiosignals in einen der sicherheitskritischen, insbesondere erstfehlersicheren Sprachbefehle basierend auf dem zweiten Sprachanalyseergebnis kann ebenfalls auf einem Vergleich eines extrahierten, zweiten Merkmalsvektors des Audiosignals mit einer Vielzahl von für jeden sicherheitskritischen Sprachbefehl spezifischen und hinterlegten Merkmalsvektoren basieren. Für jeden sicherheitskritischen, insbesondere erstfehlersicheren Sprachbefehl ist in Ausführungen der Erfindung genau ein Merkmalsvektor hinterlegt, um den Sicherheitsanforderungen für sicherheitskritische Aktionen oder Bewegungen der medizinischen Vorrichtung Rechnung zu tragen. Der Vergleich der Merkmalsvektoren kann einen individuellen Abgleich einzelner, bevorzugt aller von einem Merkmalsvektor umfassten Merkmale umfassen. Alternativ oder zusätzlich kann der Vergleich auf einem aus dem jeweiligen Merkmalsvektor abgeleiteten Merkmalsparameter basieren, welche eine Untermenge oder alle Einzelmerkmale berücksichtigt. Das sich bei dem Vergleich ergebende Übereinstimmungsmaß für die Merkmalsvektoren oder Merkmalsparameter gibt an, welcher sicherheitskritische Sprachbefehl zugeordnet wird bzw., dass kein sicherheitskritischer Sprachbefehl erkannt wurde. Es wird der sicherheitskritische Sprachbefehl als zweiter Sprachbefehl zugewiesen, der die größte Ähnlichkeit bzw. eine Ähnlichkeit zum oberhalb eines festgelegten Schwellwertes hat. Im Übrigen wird auf die Ausführungen zu der ersten trainierten Funktion verwiesen.

Erkennt die zweite trainierte Funktion keinen sicherheitskritischen Sprachbefehl, erfolgt kein Vergleich des ersten und des zweiten Sprachbefehls und es wird ein Verifikationssignal erstellt, das anzeigt, dass kein zweiter Sprachbefehl vorliegt. Das Verfahren wird abgebrochen und der erste Sprachbefehl wird verworfen. Erkennt die zweite trainierte Funktion einen sicherheitskritischen Sprachbefehl, erfolgt ein Vergleich des ersten und des zweiten Sprachbefehls. Zeigt der Vergleich keine Überstimmung der beiden Sprachbefehle, wird ein Verifikationssignal erzeugt, das den ersten Sprachbefehl nicht bestätigt und dieser wird ebenfalls verworfen. Nur, wenn der Vergleich eine ausreichende Überstimmung der beiden Sprachbefehle anzeigt, wird ein Verifikationssignal erzeugt, die den ersten Sprachbefehl bestätigt.

Die zweite trainierte Funktion kann ferner darauf trainiert sein, insbesondere mit der zweiten Gruppe neuronaler Netz-Schichten Schlüsselworte, Wortfolgen oder Befehlstrigger aus dem Merkmalsvektor abzuleiten. Die zweite Gruppe neuronaler Netz-Schichten dient also dazu, dem akustischen Merkmalsvektor ein eindeutiges Schlüsselwort zuzuweisen. Dazu kann in Bezug auf einen sicherheitskritischen Sprachbefehl ein oder einige wenige Schlüsselwörter hinterlegt sein, bspw. ein oder zwei Schlüsselwörter, die einem sicherheitskritischen Sprachbefehl eindeutige zugeordnet sind.

Schlüsselworte sind insbesondere kurze Worte oder Wortfolgen mit bspw. drei oder vier Silben. Für die Schlüsselworte der einzelnen Befehlsklassen können ebenfalls spezifische akustische Merkmalsvektoren hinterlegt sein. Das neuronale Netz des zweiten Computerlinguistik-Algorithmus kann entsprechend darauf trainiert sein, insbesondere mittels der zweiten Gruppe neuronaler Netz-Schichten, einen Vergleich zwischen dem aus dem Audiosignal extrahierten Merkmalsvektor und den hinterlegten Merkmalsvektoren entsprechend der jeweils zugewiesenen Schlüsselworte durchzuführen. Der Vergleich kann dabei so ausgeführt werden, wie mit Bezug zur ersten trainierten Funktion bereits beschrieben. Wird ein Schlüsselwort in dem Audiosignal erkannt, wird der entsprechende sicherheitskritische Sprachbefehl zugeordnet. Ermittelt die zweite trainierte Funktion keine Übereinstimmung mit einem der hinterlegten Schlüsselworte, wird kein zweiter Sprachbefehl erkannt.

Während die erste trainierte Funktion darauf trainiert ist, einen möglichst breiten und vielseitigen Satz von Audiosignalen umfassend menschliche Sprache als einen Sprachbefehl auf einer vorab definierten, insbesondere großen Menge von Befehlsklassen zu erkennen, ist die weitere trainierte Funktion darauf trainiert, einige wenige Schlüsselworte in Audiosignalen zu identifizieren.

Ein wesentlicher Unterschied zwischen der ersten und der zweiten trainierten Funktion kann also insbesondere in der Art bzw. dem Umfang der Trainingsdaten liegen. Die erste trainierte Funktion wird mit einem ersten Trainingsdatensatz trainiert wird, der in Ausführungen einen breiten und vielseitigen Satz von Audiosignalen umfassend menschliche Sprache und die jeweils zugeordneten, verschiedenen Sprachbefehlen entsprechend einer Vielzahl verschiedener Befehlsklassen umfass. Der erste Trainingsdatensatz kann auch keiner Befehlsklasse zuordenbaren Spracheingaben umfassen. Die zweite trainierte Funktion wird hingegen mit einem zweiten Trainingsdatensatz trainiert, der auf spezifische, konkret sicherheitskritische, insbesondere erstfehlersichere, also phonetisch, lexikalisch und/oder strukturell eindeutige, nur schwer verwechselbare Sprachbefehle beschränkt ist. Der zweite Trainingsdatensatz umfasst folglich in Ausführungen verschiedene Audiosignale sowie die jeweils entsprechenden sicherheitskritischen Sprachbefehle. Insbesondere ist in Ausführungen der zweite Trainingsdatensatz eine Untermenge des ersten Trainingsdatensatzes, wobei die Untermenge in Ausführungen nur erstfehlersichere Sprachbefehle betreffen kann. Insofern kann erfindungsgemäß die zweite trainierte Funktion mittels eines kleinen Trainings-Wortschatz (engl. small vocabulary) und im Gegensatz dazu die erste trainierte Funktion mit einem großen Trainings-Wortschatz (engl. large vocabulary) trainiert werden.

Ein weiterer Unterschied zwischen erster und zweiter trainierter Funktion kann in der Ausbildung der Verifikationsfunktion bzw. der Klassifizierungsfunktion liegen, die bevorzugt mittels der zweiten Gruppe neuronaler Netz-Schichten ausgeführt wird. Während die erste trainierte Funktion ausgebildet ist, eine Spracheingabe in eine breite Vielzahl verschiedener Sprachbefehle entsprechend einer großen Anzahl verschiedener Kategorien zu klassifizieren, ist die zweite trainierte Funktion ausgebildet, eine Spracheingabe in nur eine kleine Menge sicherheitskritischer Sprachbefehle entsprechend einer geringen Anzahl von Kategorien zu klassifizieren.

Insbesondere unterscheiden sich erfindungsgemäß die erste trainierte Funktion und die zweite trainierte Funktion in der Art bzw. dem Typ des jeweils eingesetzten neuronalen Netzes. Derart kann bspw. das Risiko für das Auftreten ähnlicher systematischer Fehler bei der Sprachbefehlserkennung durch die erste trainierte Funktion und die zweite trainierte Funktion reduziert werden.

Besonders bevorzugt kann bei der ersten trainierten Funktion auf an sich bekannte und verfügbare Spracherkennungs-Algorithmen zurückgegriffen werden. In Implementierungen der Erfindung entspricht die zweite trainierte Funktion einem im Rahmen eines sicheren, bspw. herstellerseitigen Software-Entwicklungsprozesses erzeugten Spracherkennungs-Algorithmus.

Während die erste trainierte Funktion bspw. als ein Feedforward-Netz ausgebildet sein kann, kann die zweite trainierte Funktion bspw. als rekurrentes bzw. rückgekoppeltes Netz ausgebildet sein, bei dem Knoten einer Schicht auch mit sich selbst oder mit anderen Knoten derselben und/oder wenigstens einer vorangegangenen Schicht verknüpft sind.

Gemäß einigen Implementierungen kann der erste Computerlinguistik-Algorithmus als sog. Front-End-Algorithmus implementiert sein, der z.B. in einer lokalen Recheneinheit der medizinischen Vorrichtung oder in einem lokalen Spracherkennungsmodul, gehostet ist. Als Front-End kann die Verarbeitung insbesondere gut in Echtzeit erfolgen, sodass das Ergebnis praktisch ohne nennenswerte Zeitverzögerung erhalten werden kann. Entsprechend kann der zweite Computerlinguistik-Algorithmus als sog. Back-End-Algorithmus implementiert sein, der z.B. in einer entfernten Recheneinrichtung, wie etwa einem reellen Server-basierten Rechensystem oder einem virtuellen Cloud-Rechensystem, gehostet ist. In einer Back-End-Implementierung können insbesondere komplexe Analysealgorithmen eingesetzt werden, die eine hohe Rechenleistung erfordern. Entsprechend kann das Verfahren ein Übermitteln des Audiosignals an eine entfernte Recheneinrichtung und ein Empfangen ein oder mehrerer Analyseergebnisse von der entfernten Recheneinrichtung umfassen. In alternativen Implementierungen kann auch der zweite Computerlinguistik-Algorithmus als Front-End-Algorithmus implementiert sein. Umgekehrt kann auch der erste Computerlinguistik-Algorithmus als Back-End-Algorithmus implementiert sein.

Gemäß einer Vielzahl bevorzugter Implementierungen der Erfindung umfasst ein Analysieren des Audiosignals ein Tokenisieren zur Segmentierung von Buchstaben, Wörtern und/oder Sätzen innerhalb des Audiosignals mittels des ersten und des zweiten Computerlinguistik-Algorithmus. Der erste und der zweite Sprachbefehl werden dabei basierend auf dem eine erste Tokenisierungsinformation umfassenden ersten Sprachanalyseergebnis und dem eine zweite Tokenisierungsinformation umfassenden zweiten Sprachanalyseergebnis erkannt.

In Ausführungen der Erfindung kann auch nur der erste oder der zweite Computerlinguistik-Algorithmus ein Tokenisieren ausführen. Insbesondere kann die jeweils eine erste Netzschicht der ersten und/oder zweiten trainierten Funktion ausgebildet sein, das Audiosignal zu tokenisieren.

Tokenisierung bezeichnet in der Computerlinguistik die Segmentierung eines Textes in Einheiten der Buchstaben-, Wort- und Satzebene. Gemäß einigen Implementierungen kann das Tokenisieren ein Umwandeln der im Audiosignal enthaltenen Sprache in Text umfassen. Mit anderen Worten kann ein Transskript erstellt werden, das dann tokenisiert wird. Dazu kann auf eine Vielzahl an sich bekannter Verfahren zurückgegriffen werden, die z.B. auf einer Verwendung einer Formantanalyse, von Hidden-Markov-Modellen, neuronalen Netzen, elektronischen Wörterbüchern und/oder Sprachmodellen beruhen. Bevorzugt wird dieser Analyseschritt mittels der ersten, zweiten und/oder der weiteren trainierten Funktion, wie eingangs beschrieben, ausgeführt.

Das erste und/oder zweite Sprachanalyseergebnis kann eine erste bzw. zweite Tokenisierungsinformation umfassen. Durch die Verwendung der Tokenisierungsinformation kann die Struktur einer Spracheingabe für die Ermittlung eines Sprachbefehls bzw. allgemein einer Nutzerintention berücksichtigt werden.

Gemäß einiger besonders bevorzugter Implementierungen umfasst das Analysieren des Audiosignals eine semantische Analyse des Audiosignals mittels des ersten und des zweiten Computerlinguistik-Algorithmus. Der erste und der zweite Sprachbefehl werden dabei basierend auf dem eine erste semantische Information umfassenden ersten Sprachanalyseergebnis und dem eine zweite semantische Information umfassenden zweiten Sprachanalyseergebnis identifiziert werden.

In Ausführungen der Erfindung kann auch nur der erste oder der zweite Computerlinguistik-Algorithmus eine semantische Analyse des Audiosignals ausführen. Insbesondere kann die jeweils eine erste Netzschicht der ersten und/oder zweiten trainierten Funktion ausgebildet sein, das Audiosignal semantisch zu analysieren.

Die semantische Analyse stellt darauf ab, die Bedeutung der Spracheingabe der Bedienperson zu erschließen. Insbesondere kann die semantische Analyse einen vorgelagerten Spracherkennungsschritt (speech to text) und oder einen Tokenisierungsschritt umfassen.

Gemäß einigen Implementierungen zeigt die semantische Information an, ob das Audiosignal ein oder mehrere Nutzerintentionen enthält oder nicht. Die Nutzerintention kann dabei insbesondere eine auf einen oder mehrere in Frage kommende Sprachbefehle gerichtete Spracheingabe der Bedienperson sein. Die Sprachbefehle können dabei insbesondere zur Steuerung der medizinischen Vorrichtung relevante Sprachbefehle sein. Gemäß einigen Implementierungen indiziert bzw. enthält die semantische Information wenigstens eine Eigenschaft einer in dem Audiosignal enthaltenen Nutzerintention. Mit der semantischen Analyse werden also bestimmte akustische Charakteristika bzw. Eigenschaften aus der Spracheingabe extrahiert, die für die Bestimmung eines Sprachbefehls berücksichtigt werden bzw. relevant sein können.

In weiteren Implementierungen umfasst ein erfindungsgemäßes Verfahren auch ein Klassifizieren wenigstens des ersten Sprachbefehls in eine Sicherheitsklasse aus einer Vielzahl von Sicherheitsklassen, wobei wenigstens eine der mehreren Sicherheitsklassen für sicherheitskritische Sprachbefehle vorgesehen ist. Das Ermitteln des Verifikationssignals für den ersten Sprachbefehl wird hier ausgeführt, wenn dieser der Sicherheitsklasse für sicherheitskritische Sprachbefehle zugeordnet wurde. Der Verifikationsschritt wird also bevorzugt ausgeführt, wenn der erste Sprachbefehl entsprechend einer Sicherheitsklasse für sicherheitskritische Sprachbefehle klassifiziert wurde. Wie eingangs erwähnt, umfassen sicherheitskritische Sprachbefehle in Implementierungen Sprachbefehle deren Ausführung durch die medizinische Vorrichtung, insbesondere erstfehlersicher ausgebildet werden müssen.

Die Zuordnung eines Sprachbefehls bzw. einer Befehlsklasse zu einer Sicherheitsklasse erfolgt in Implementierungen basierend auf einer vorab festgelegten Zuordnungsvorschrift, die die Art des Sprachbefehls bzw. ein Gefährdungsmaß der durch den Sprachbefehl auszulösenden Operation bzw. Aktion bzw. Bewegung der medizinischen Vorrichtung für den Patienten und/oder das Bedienpersonal und/oder die medizinische Vorrichtung oder für anderes medizinisches Equipment berücksichtigt. In Implementierungen erfolgt die Zuordnung über eine vorab festgelegte Lookup-Tabelle. In weiteren Implementierungen können vorab definierte Schlüsselwörter vorgesehen sein, die von dem ersten Computerlinguistik-Algorithmus in dem Audiosignal erkannt werden können, die jeweils mit einer der Sicherheitsklassen verknüpft sind. Einer Sicherheitsklasse können dabei ein oder mehrere Schlüsselwörter zugeordnet sein. Dem ersten Sprachbefehl wird in Ausführungen also eine Sicherheitsklasse zugeordnet, wenn ein oder mehrere Schlüsselwörter durch den ersten Computer-Linguistik-Algorithmus in dem Audiosignal erkannt wurden. Alternativ kann die Sicherheitsklasse bei Erkennung des ersten Sprachbefehls automatisch per vorab definierter Zuordnungsvorschrift festgelegt sein.

Der Schritt des Zuordnens des ersten Sprachbefehls zu einer Sicherheitsklasse ist folglich darauf gerichtet, den ersten Sprachbefehl als sicherheitskritischen, insbesondere als erstfehlersicheren, oder nicht sicherheitskritischen Sprachbefehl zu identifizieren. In Implementierungen wird auch dieser Schritt durch den ersten Computerlinguistik-Algorithmus, bevorzugt die erste trainierte Funktion, ausgeführt. Die erste trainierte Funktion kann also darauf trainiert sein, eine Sicherheitsklasse zu dem ersten Sprachbefehl zuzuordnen. Dann können die Ausgabedaten der trainierten Funktion des ersten Computerlinguistik-Algorithmus auch die Sicherheitsklasse umfassen. Insbesondere kann eine dritte Gruppe neuronaler Netz-Schichten ausgebildet sein, basierend auf der Befehlsklasse und/oder dem erkannten ersten Sprachbefehl eine Sicherheitsklasse zuzuordnen, wobei insbesondere die ermittelte Befehlsklasse und/oder der erste Sprachbefehl als Eingabe-Daten für die dritte Gruppe neuronaler Netz-Schichten dienen. Die dritte Gruppe ist nun ausgebildet, die Befehlsklasse und/oder den erkannten Sprachbefehl als sicherheitskritischen, insbesondere erstfehlersicheren Befehl oder nicht sicherheitskritischen Befehl zu klassifizieren.

In Ausführungen der Erfindung ist das Erkennen des ersten Sprachbefehls als sicherheitskritischer Sprachbefehl Voraussetzung für die Ermittlung eines Verifikationssignals.

In anderen bevorzugten Implementierungen umfasst das Erfassen des Audiosignals ein Erfassen des Audiosignals mittels einer ersten Eingabevorrichtung und einer zweiten Eingabevorrichtung, wobei sich die erste und die zweite Eingabevorrichtung voneinander unterscheiden. Das Erfassen umfasst hier auch ein Bereitstellen des mittels der ersten Eingabevorrichtung erfassten Audiosignals für das Analysieren und Bereitstellen des ersten Sprachanalyseergebnisses, und ein Bereitstellen des mittels der zweiten Eingabevorrichtung erfassten Audiosignals für das Analysieren und Bereitstellen des zweiten Sprachanalyseergebnisses.

In dieser Ausführung wird das Audiosignal mittels zweier redundanter Eingabevorrichtungen, bspw. zwei unabhängigen Mikrophonen erfasst. Fehler, die bei der Signalerfassung, also bei der Registrierung, der Digitalisierung und/oder der Speicherung der Spracheingabe in einer der Eingabevorrichtungen entstehen können, können sich dadurch nur in die Ableitung des ersten oder des zweiten Sprachbefehls fortpflanzen, mit großer Wahrscheinlichkeit aber nicht in beiden.

Gemäß einem weiteren Aspekt stellt die Erfindung eine Sprachsteuervorrichtung zur Sprachsteuerung einer medizinischen Vorrichtung bereit. Die Sprachsteuervorrichtung umfasst wenigstens eine Schnittstelle zum Erfassen eines Audiosignals enthaltend eine auf die Steuerung der Vorrichtung gerichtete Spracheingabe einer Bedienperson. Die Sprachsteuervorrichtung umfasst ferner eine erste Auswerteeinheit, die ausgebildet ist, das Audiosignal zu analysieren und ein erstes Sprachanalyseergebnis bereitzustellen und einen ersten Sprachbefehl basierend auf dem ersten Sprachanalyseergebnis zu erkennen. Die erste Auswerteeinheit kann auch ausgebildet sein, den ersten Sprachbefehl zu einer Sicherheitsklasse zuzuordnen, wobei wenigstens eine Sicherheitsklasse für sicherheitskritische Sprachbefehle vorgesehen ist. Die Sprachsteuervorrichtung umfasst auch eine zweite Auswerteeinheit, die ausgebildet ist, ein Verifikationssignal für den ersten Sprachbefehl zu ermitteln und für eine Weiterverarbeitung bereitzustellen. Die zweite Auswerteeinheit ist insbesondere ausgebildet das Audiosignal zum Bereitstellen eines zweiten Sprachanalyseergebnisses zu analysieren, einen zweiten Sprachbefehl der Bedienperson basierend auf dem zweiten Sprachanalyseergebnis zu erkennen und den ersten und den zweiten Sprachbefehls zu vergleich, wobei das Verifikationssignal den ersten Sprachbefehl bestätigt, wenn der erste Sprachbefehl und der zweite Sprachbefehl ein Übereinstimmungskriterium erfüllen. Die Sprachsteuervorrichtung umfasst weiter eine Steuereinheit, die ausgebildet ist, ein Steuersignal zur Steuerung der medizinischen Vorrichtung basierend auf dem Verifikationssignal und ggf. dem ersten Sprachbefehl und zu erzeugen, sofern der erste Sprachbefehl anhand der Verifikationsinformation bestätigt wurde. Dabei ist das Steuersignal geeignet, die medizinische Vorrichtung entsprechend dem ersten Sprachbefehl zu steuern. Die Sprachsteuervorrichtung umfasst auch eine Schnittstelle zur Eingabe des Steuersignals in die medizinische Vorrichtung. Beide Schnittstellen können als eine integrale Ein- und Ausgabeschnittstelle ausgebildet sein.

Eine medizinische Vorrichtung im Sinne der Erfindung ist insbesondere eine gegenständliche medizinische Vorrichtung. Die medizinische Vorrichtung wird typischerweise zur Behandlung und/oder Untersuchung eines Patienten eingesetzt. Die medizinische Vorrichtung kann insbesondere dazu ausgebildet sein, eine medizinische Prozedur durchzuführen und/oder zu unterstützen. Die medizinische Prozedur kann eine bildgebende und/oder interventionelle und/oder therapeutische Prozedur aber auch eine Überwachung eines Patienten umfassen. Insbesondere kann die medizinische Vorrichtung eine bildgebende Modalität umfassen, wie beispielsweise ein Magnetresonanzgerät, ein Einzelphotonenemissionstomographie-Gerät (SPECT-Gerät), ein Positronen-Emissions-Tomographie-Gerät (PET-Gerät), ein Computertomograph, ein Ultraschall-Gerät, ein Röntgengerät oder ein als C-Bogen-Gerät ausgebildetes Röntgengerät. Die bildgebende Modalität kann auch ein kombiniertes medizinisches Bildgebungsgerät sein, welches eine beliebige Kombination aus mehreren der genannten bildgebenden Modalitäten umfasst. Ferner kann die medizinische Vorrichtung eine Interventions- und/oder Therapievorrichtung aufweisen, wie etwa eine Biopsievorrichtung, eine Strahlen- bzw. Radiotherapievorrichtung zur Bestrahlung eines Patienten, und/oder eine Eingriffsvorrichtung zur Durchführung eines, insbesondere minimalinvasiven, Eingriffs. Gemäß weiterer Implementierungen umfasst die medizinische Vorrichtung zusätzlich oder alternativ Patientenüberwachungsmodule, wie etwa ein EKG-Gerät, und/oder ein Patienten-Pflegegerät umfassen, wie etwa ein Beatmungsgerät, ein Infusionsgerät und/oder ein Dialysegerät. Die Behandlung und/oder Untersuchung und/oder Überwachung des Patienten mittels der medizinischen Vorrichtung wird in Ausführungen typischerweise von einer Bedienperson, beispielsweise Pflegepersonal, technischem Personal, Röntgenassistenzpersonal oder Ärztinnen, unterstützt bzw. gesteuert.

Die erste und zweite Auswerteeinheit sowie die Steuereinheit können als eine oder mehrere zentrale und/oder dezentrale Recheneinheiten ausgebildet sein. Die Recheneinheit(en) kann/können jeweils einen oder mehrere Prozessoren aufweisen. Ein Prozessor kann als zentrale Verarbeitungseinheit (CPU/GPU) ausgebildet sein. Insbesondere kann die erste und/oder die zweite Auswerteeinheit sowie die Steuereinheit jeweils als Teil oder Modul einer, durch Spracheingabe zu steuernden, medizinischen Vorrichtung implementiert sein. In Implementierungen können die Auswerteeinheiten jeweils als Submodul der Steuereinheit ausgebildet sein oder umgekehrt. Alternativ kann die wenigstens eine Auswerteeinheit als lokaler oder Cloud-basierter Verarbeitungsserver implementiert sein. Ferner kann die wenigstens eine Auswerteeinheit ein oder mehrere virtuelle Maschinen umfassen.

In bevorzugter Implementierung sind die erste und die zweite Auswerteeinheit als voneinander getrennte Recheneinheiten ausgebildet. Das heißt, die beiden Auswerteeinheiten, sind jeweils wie eingangs beschrieben auf separater Hardware bzw. in verschiedenen Software-Modulen implementiert. Derart ist die Sprachsteuervorrichtung eingerichtet, die klassische Control-Protect (CP) -Struktur eines erstfehlersicheren Steuerungssystems für Steuerbefehle in Form von Spracheingabe abzubilden, in welchem die erste Auswerteinheit einen Teil des Steuer-Pfades (C-Pfad) und die zweite Auswerteinheit den Prüf-Pfad (P-Pfad) ausbildet.

Die Schnittstelle der Sprachsteuervorrichtung kann allgemein zum Datenaustausch zwischen der Sprachsteuerungsvorrichtung und weiteren Komponenten und oder zum Datenaustausch von Komponenten oder Modulen der Sprachsteuervorrichtung untereinander ausgebildet sein. Die Schnittstelle kann insofern in Form von einer oder mehreren einzelnen Datenschnittstellen implementiert sein, welche ein Hardware- und/oder Software-Interface, z.B. einen PCI-Bus, eine USB-Schnittstelle, eine Fire-Wire-Schnittstelle, eine Zig-Bee- oder eine Bluetooth-Schnittstelle aufweisen können. Die Schnittstelle kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend können die ein oder mehreren Datenschnittstellen eine LAN-Schnittstelle oder eine Wireless LAN-Schnittstelle (WLAN oder Wi-Fi) aufweisen. Die Schnittstelle kann ferner zur Kommunikation mit der Bedienperson über eine Benutzerschnittstelle ausgebildet sein. Entsprechend kann die Schnittstelle dazu ausgebildet sein, Sprachbefehle über die Benutzerschnittstelle anzuzeigen und diesbezügliche Nutzereingaben über die Benutzerschnittstelle zu empfangen. Insbesondere kann die Schnittstelle eine akustische Eingabevorrichtung zur Registrierung des Audiosignals und in Ausführungen eine akustische Ausgabevorrichtung zur Ausgabe eines eine Aufforderung zur Bestätigung des ersten Sprachbefehls umfassenden Audiosignals umfassen.

In weiterer bevorzugter Implementierung umfasst die Schnittstelle eine erste und eine zweite Eingabevorrichtung zum gleichzeitigen Erfassen des Audiosignals. Systematische oder zufällige Fehler bei der Audiosignalerfassung werden auf einen der Auswertungspfade zur Ermittlung des ersten und des zweiten Sprachbefehls beschränkt.

Die Vorteile der vorgeschlagenen Vorrichtung entsprechen im Wesentlichen den Vorteilen des vorgeschlagenen Verfahrens. Merkmale, Vorteile oder alternative Ausführungsformen/Aspekte können ebenso auf die anderen beanspruchten Gegenstände übertragen werden und umgekehrt.

Gemäß einem weiteren Aspekt stellt die Erfindung ein medizinisches System umfassend die erfindungsgemäße Sprachsteuervorrichtung und eine medizinische Vorrichtung zur Durchführung einer medizinischen Prozedur bereit.

Die Erfindung betrifft in einem weiteren Aspekt ein Computerprogrammprodukt, das ein Programm umfasst und direkt in einen Speicher einer programmierbaren Recheneinheit ladbar ist und Programmmittel, bspw. Bibliotheken und Hilfsfunktionen, aufweist, um ein Verfahren zur Sprachsteuerung einer medizinischen Vorrichtung insbesondere gemäß den vorgenannten Implementierungen/Aspekten auszuführen, wenn das Computerprogrammprodukt ausgeführt wird.

Ferner betrifft die Erfindung in einem weiteren Aspekt ein computerlesbares Speichermedium, auf welchem lesbare und ausführbare Programmabschnitte gespeichert sind, um alle Schritte eines Verfahrens zur Sprachsteuerung einer medizinischen Vorrichtung gemäß den vorgenannten Implementierungen/Aspekten auszuführen, wenn die Programmabschnitte von einer Recheneinheit ausgeführt werden.

Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Recheneinheit zu laden ist. Durch das Computerprogrammprodukt können die erfindungsgemäßen Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, einen entsprechenden Prozessor oder eine entsprechende Logikeinheit aufweisen, sodass die jeweiligen Verfahrensschritte effizient ausgeführt werden können.

Das Computerprogrammprodukt ist beispielsweise auf einem computerlesbaren Speichermedium gespeichert oder auf einem Netzwerk oder Server hinterlegt, von wo es in den Prozessor der jeweiligen Recheneinheit geladen werden kann, der mit der Recheneinheit direkt verbunden oder als Teil der Recheneinheit ausgebildet sein kann. Weiterhin können Steuerinformationen des Computerprogrammprodukts auf einem computerlesbaren Speichermedium gespeichert sein. Die Steuerinformationen des computerlesbaren Speichermediums können derart ausgebildet sein, dass sie bei Verwendung des Datenträgers in einer Recheneinheit ein erfindungsgemäßes Verfahren durchführen. Beispiele für computerlesbaren Speichermedium sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen/Aspekte der vorab beschriebenen Verfahren durchgeführt werden. So kann die Erfindung auch von dem besagten computerlesbaren Medium und/oder dem besagten computerlesbaren Speichermedium ausgehen. Die Vorteile der vorgeschlagenen Computerprogrammprodukte bzw. der zugehörigen computerlesbaren Medien entsprechen im Wesentlichen den Vorteilen der vorgeschlagenen Verfahren.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet.

FIG 1 ein schematisches Blockdiagramm eines Systems zur Steuerung einer medizinischen Vorrichtung gemäß einer Ausführungsform,

FIG 2 ein weiteres Blockdiagramm eines Systems zur Steuerung einer medizinischen Vorrichtung gemäß einer weiteren Ausführungsform,

FIG 3 ein schematisches Ablaufdiagramm eines Verfahrens zur Steuerung einer medizinischen Vorrichtung gemäß einer Ausführungsform,

FIG 4 ein weiteres schematisches Ablaufdiagramm eines Verfahrens zur Steuerung einer medizinischen Vorrichtung gemäß einer Ausführungsform, und

FIG 5 ein neuronales Netz eines erfindungsgemäßen Computerlinguistik-Algorithmus in einer Ausführungsform.

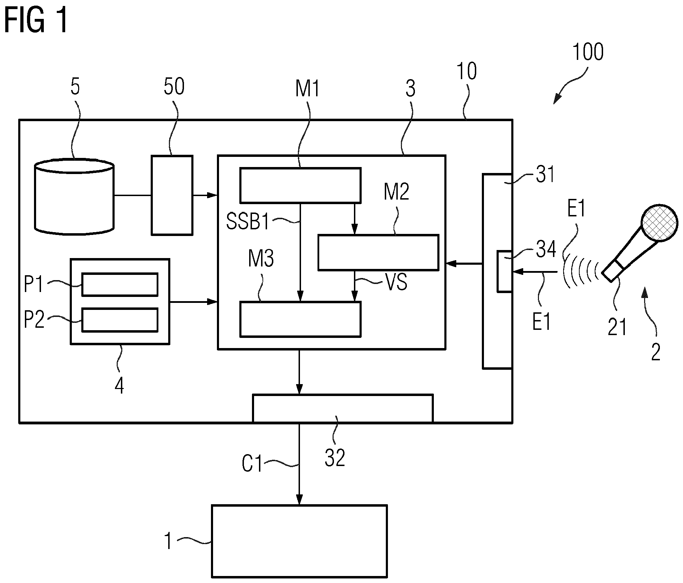

Figur 1 zeigt schematisch eine funktionale Blockdarstellung eines Systems 100 zur Steuerung einer medizinischen Vorrichtung 1. Das System 100 umfasst die medizinische Vorrichtung 1, die zur Durchführung einer medizinischen Prozedur an einem Patienten ausgebildet ist. Die medizinische Prozedur kann eine bildgebende und/oder interventionelle und/oder therapeutische Prozedur umfassen. Das System umfasst ferner eine Sprachsteuervorrichtung 10.

Die medizinische Vorrichtung 1 kann eine bildgebende Modalität umfassen. Die bildgebende Modalität kann allgemein dazu ausgebildet sein, einen anatomischen Bereich eines Patienten abzubilden, wenn dieser in einen Erfassungsbereich der bildgebenden Modalität gebracht wird. Die bildgebende Modalität ist beispielsweise ein Magnetresonanzgerät, ein Einzelphotonenemissionstomographie-Gerät (SPECT-Gerät), ein Positronen-Emissions-Tomographie-Gerät (PET-Gerät), ein Computertomograph, ein Ultraschall-Gerät, ein Röntgengerät oder ein als C-Bogen-Gerät ausgebildetes Röntgengerät. Die bildgebende Modalität kann auch ein kombiniertes medizinisches Bildgebungsgerät sein, welches eine beliebige Kombination aus mehreren der genannten bildgebenden Modalitäten umfasst.

Ferner kann die medizinische Vorrichtung 1 eine Interventions- und/oder Therapievorrichtung aufweisen. Die Interventions- und/oder Therapievorrichtung kann allgemein dazu ausgebildet sein, eine interventionelle und/oder therapeutische medizinische Prozedur an dem Patienten durchzuführen. Beispielsweise kann die Interventions- und/oder Therapievorrichtung eine Biopsievorrichtung zur Entnahme einer Gewebeprobe, eine Strahlen- bzw. Radiotherapievorrichtung zur Bestrahlung eines Patienten, und/oder eine Eingriffsvorrichtung zur Durchführung eines, insbesondere minimalinvasiven, Eingriffs sein. Gemäß Ausführungsformen der Erfindung kann die Interventions- und/oder Therapievorrichtung automatisiert oder wenigstens teil-automatisiert sowie insbesondere robotergesteuert sein. Die Strahlen- bzw. Radiotherapievorrichtung kann beispielsweise einen medizinischen Linearbeschleuniger oder eine andere Strahlenquelle aufweisen. Beispielsweise kann die Eingriffsvorrichtung einen Katheterroboter, minimalinvasiven chirurgischen Roboter, einen Endoskopieroboter usw. aufweisen.

Gemäß weiteren Ausführungsformen kann die medizinische Vorrichtung 1 zusätzlich oder alternativ Einheiten und/oder Module aufweisen, welche die Durchführung einer medizinischen Prozedur unterstützen, wie etwa eine insbesondere wenigstens teil-automatisiert steuerbare Patientenlagerungsvorrichtung und/oder Überwachungsgeräte zur Überwachung eines Zustands des Patienten, wie etwa ein EKG-Gerät, und/oder ein Patientenversorgungsgerät, wie etwa ein Beatmungsgerät, ein Infusionsgerät und/oder ein Dialysegerät.

Ein oder mehrere Komponenten der medizinischen Vorrichtung 1 sollen gemäß Ausführungsformen der Erfindung durch eine oder mehrere Spracheingaben einer Bedienperson ansteuerbar sein. Dazu weist das System 100 eine Sprachsteuervorrichtung 10 umfassend eine Schnittstelle mit akustischer Eingabevorrichtung 2 auf.

Die akustische Eingabevorrichtung 2 dient zum Aufnehmen oder Erfassen eines Audiosignals E1, also zum Aufnehmen gesprochener Laute, die von einer Bedienperson des Systems 100 erzeugt werden. Die Eingabevorrichtung 2 kann beispielsweise als Mikrophon realisiert sein. Die Eingabevorrichtung 2 kann beispielsweise an der medizinische Vorrichtung 1 oder an anderer Stelle, wie in einem entfernten (engl. remote) Bedienraum, stationär angeordnet sein. Alternativ kann die Eingabevorrichtung 2 auch portabel realisiert sein, z.B. als Mikrophon eines Headsets, das von der Bedienperson mitführbar ist. In diesem Fall weist die Eingabevorrichtung 2 vorteilhaft einen Sender 21 zur drahtlosen Datenübertragung auf.

Die Sprachsteuervorrichtung 10 weist einen Eingang 31 zum Empfang von Signalen und einen Ausgang 32 zum Bereitstellen von Signalen auf. Der Eingang 31 und der Ausgang 32 können eine Schnittstelleneinrichtung der Sprachsteuervorrichtung 10 bilden. Die Sprachsteuervorrichtung 10 ist allgemein zur Durchführung von Datenverarbeitungsprozessen und zur Erzeugung elektrischer Signale eingerichtet.

Hierzu kann die Sprachsteuervorrichtung 10 wenigstens eine Recheneinheit 3 aufweisen. Die Recheneinheit 3 kann z.B. einen Prozessor, z.B. in Form einer CPU oder dergleichen umfassen. Die Recheneinheit 3 weist bevorzugt eine erste und eine zweite zentrale Auswerteeinheit auf, z.B. jeweils als eine Auswerteeinheit mit einem oder mehreren Prozessoren. Die Recheneinheit 3 kann bevorzugt eine Steuereinheit umfassen, die ausgebildet ist, Steuersignale für die medizinische Vorrichtung zu erzeugen.

Die Recheneinheit 3 kann insbesondere zumindest teilweise als Steuerungsrechner (engl. System control) der medizinischen Vorrichtung 1 oder als ein Teil desselben ausgebildet sein. Die Recheneinheit 3 umfasst erfindungsgemäß Einheiten bzw. Module, die zur Ausführung einer Sicherheitsfunktion, insbesondere genormten Sicherheitsfunktion (auch SIL (engl. safety integrity level)-Einheiten) ausgebildet sind. Die genormte Sicherheitsfunktion dient dazu, ein Betriebsrisiko der medizinischen Vorrichtung 1 durch Ausführung eines fälschlich erkannten Steuerbefehls zu minimieren. Insbesondere ermöglicht die Sicherheitsfunktion die Absicherung eines Erstfehlers bei der maschinellen Erkennung eines Steuerbefehls aus einer Spracheingabe. Gemäß weiteren Implementierungen können Funktionalitäten und Komponenten der Recheneinheit 3 in einer dezentralisierten Weise über mehrere Recheneinheiten oder Steuermodule des Systems 100 verteilt sein.

Ferner weist die Sprachsteuervorrichtung 10 wenigstens einen Datenspeicher 4 auf, und zwar insbesondere einen durch die Recheneinheit 3 lesbaren, nichtflüchtigen Datenspeicher wie eine Festplatte, eine CD-ROM, eine DVD, eine Blu-Ray Disc, eine Diskette, einen Flash-Speicher oder dergleichen. Auf dem Datenspeicher 4 kann allgemein Software P1, P2 gespeichert sein, die dazu eingerichtet ist, die Recheneinheit 3 zur Durchführung der Schritte eines Verfahrens zu veranlassen.

Wie in Figur 1 schematisch dargestellt ist, ist der Eingang 31 der Sprachsteuervorrichtung 10 mit der Eingabevorrichtung 2 verbunden. Der Eingang kann ebenfalls mit der medizinischen Vorrichtung 1 verbunden sein. Der Eingang 31 kann zur drahtlosen oder zur drahtgebundenen Datenkommunikation eingerichtet sein. Beispielsweise kann der Eingang 31 einen Bus-Anschluss aufweisen. Alternativ oder zusätzlich zu einem drahtgebundenen Anschluss kann der Eingang 31 auch eine Schnittstelle, z.B. einen Empfänger 34 zur drahtlosen Datenübertragung aufweisen. Beispielsweise kann, wie in Figur 1 dargestellt, der Empfänger 34 mit dem Sender 21 der Eingabevorrichtung 2 in Datenkommunikation stehen. Als Empfänger 34 kann beispielsweise eine WIFI Schnittstelle, eine Bluetooth-Schnittstelle oder dergleichen vorgesehen sein.

Der Ausgang 32 der Sprachsteuervorrichtung 10 ist einerseits mit der medizinischen Vorrichtung 1 verbunden. Der Ausgang 32 kann zur drahtlosen oder zur drahtgebundenen Datenkommunikation eingerichtet sein. Beispielsweise kann der Ausgang 32 einen Bus-Anschluss aufweisen. Alternativ oder zusätzlich zu einem drahtgebundenen Anschluss kann der Ausgang 32 auch eine Schnittstelle zur drahtlosen Datenübertragung, bspw. zu einem Online-Modul OM1, aufweisen, beispielsweise eine WIFI Schnittstelle, eine Bluetooth-Schnittstelle oder dergleichen.

Die Sprachsteuervorrichtung 10 ist dazu eingerichtet, ein oder mehrere Steuersignale C1 zur Steuerung der medizinischen Vorrichtung 1 zu erzeugen und an dem Ausgang 32 bereitzustellen. Das Steuerkommando C1 veranlasst die medizinische Vorrichtung 1 zur Durchführung eines bestimmten Arbeitsschritts oder einer Sequenz von Schritten. Am Beispiel einer als MR-Gerät ausgeführten bildgebenden Modalität können sich solche Schritte beispielsweise auf die Durchführung einer bestimmten Scansequenz mit einer bestimmten Anregung von Magnetfeldern durch einen Erzeugerkreis des MR-Geräts beziehen. Ferner können sich solche Schritte auf ein Verfahren von verfahrbaren Systemkomponenten der medizinischen Vorrichtung 1 beziehen, wie etwa dem Verfahren einer Patientenlagerungsvorrichtung oder dem Verfahren von Emissions- oder Detektor-Komponenten einer bildgebenden Modalität. Die Schritte können sich insbesondere auch auf das Auslösen bzw. Starten einer Röntgenstrahlung beziehen.

Zur Bereitstellung des Steuersignals C1 kann die Recheneinheit 3 verschiedene Module M1-M3 aufweisen. Ein erstes Modul M1 entsprechend einer ersten Auswerteeinheit ist dazu ausgebildet, das Audiosignal E1 zu analysieren und basierend drauf ein erstes Sprachanalyseergebnis bereitzustellen, einen ersten Sprachbefehl SSB1 basierend auf dem ersten Sprachanalyseergebnis zu erkennen. Modul M1 kann in Ausführungen auch ausgebildet sein, den ersten Sprachbefehl SSB1 zu einer Sicherheitsklasse SK zuzuordnen. Hierfür kann das Modul M1 dazu ausgebildet sein, einen ersten Computerlinguistik-Algorithmus P1 auf das Audiosignal E1 anzuwenden. Insbesondere ist das Modul M1 ausgebildet, die Verfahrensschritte S20 bis S40 auszuführen.

Der erste Sprachbefehl SSB1 kann anschließend in ein weiteres Modul M2 entsprechend einer zweiten, von der ersten unabhängigen Auswerteeinheit eingegeben werden. Insbesondere wird der erste Sprachbefehl SSB1 dann an Modul M2 weitergeben, wenn er als sicherheitskritischer, insbesondere erstfehlersicherer Sprachbefehl erkannt wurde (bspw. durch den Schritt des Zuordnens zu einer Sicherheitsklasse SK umfassend sicherheitskritische Sprachbefehle). Modul M2 ist dazu ausgebildet, ein Verifikationssignal VS zur Bestätigung des ersten Sprachbefehls SSB1 zu ermitteln. Dazu kann das Modul M2 ausgebildet sein, einen zweiten Computerlinguistik-Algorithmus P2 auf das Audiosignal E1 anzuwenden. Der zweite Computerlinguistik-Algorithmus P2 kann dabei ausgebildet sein, einen zweiten Sprachbefehl SSB2 in dem Audiosignal E1 zu erkennen und einen Vergleich zwischen erstem und zweiten Sprachbefehl SSB1 und SSB2 durchzuführen. Der zweite Computerlinguistik-Algorithmus P2 kann auch ausgebildet sein, wenigstens ein Schlüsselwort bzw. eine spezifische Wortfolge in dem zweiten Audiosignal E2 zu erkennen, welche(s) mit einem sicherheitskritischen Sprachbefehl verknüpft sein kann.

Das zweite Modul M2 ist insbesondere ausgebildet, basierend auf einem Vergleich zwischen erstem und zweitem Sprachbefehl SSB1, SSB2 ein Verifikationssignal VS umfassend eine Verifikationsinformation, bestätigend den ersten Sprachbefehl SSB1 zu erzeugen. Das Verifikationssignal VS kann bevorzugt auch den ersten Sprachbefehl SSB1 umfassen.

Das Verifikationssignal VS wird an ein drittes Modul M3 entsprechend einer Steuereinheit der Sprachsteuervorrichtung 10 gegeben. Modul M3 ist dazu ausgebildet, basierend auf dem Verifikationssignal VS und ggf. dem ersten Sprachbefehl SSB1 ein oder mehrere Steuersignale C1 bereitzustellen, die dazu geeignet sind, die medizinische Vorrichtung 1 entsprechend dem ersten Sprachbefehl SSB1 zu steuern.

Gehört der erste Sprachbefehl SSB1 einer Sicherheitsklasse SK betreffend nicht sicherheitskritische Sprachbefehle an, wird in Ausführungen das Modul M2 nicht aktiviert. Modul M1 gibt dann den erkannten ersten Sprachbefehl SSB1 bspw. direkt in das Modul M3 ein. Eine erfindungsgemäße Verifikation kann hier entfallen.

Die vorgenommene Unterteilung in Module M1-M3 dient dabei lediglich der einfacheren Erklärung der Funktionsweise der Recheneinheit 3 und ist nicht beschränkend zu verstehen. Die Module M1-M3 können dabei insbesondere auch als Computerprogrammprodukte oder Computerprogrammsegmente aufgefasst werden, welche bei der Ausführung in der Recheneinheit 3 ein oder mehrere der nachstehend beschriebenen Funktionen oder Verfahrensschritte realisieren.

Bevorzugt ist mindestens Modul M2 als eine SIL-Einheit zur Ausführung einer Sicherheitsfunktion ausgebildet. Die Sicherheitsfunktion ist erfindungsgemäß darauf gerichtet, den ersten erkannten Sprachbefehl SSB1 in wenigstens einer unabhängigen Verifikationsschleife zu prüfen bzw. zu bestätigen.

Entsprechend einer klassischen CP-Struktur bilden Modul M1 und Modul M3 zusammen einen Teil des C-Pfades aus, während Modul M2 einen Teil des P-Pfades ausbildet.

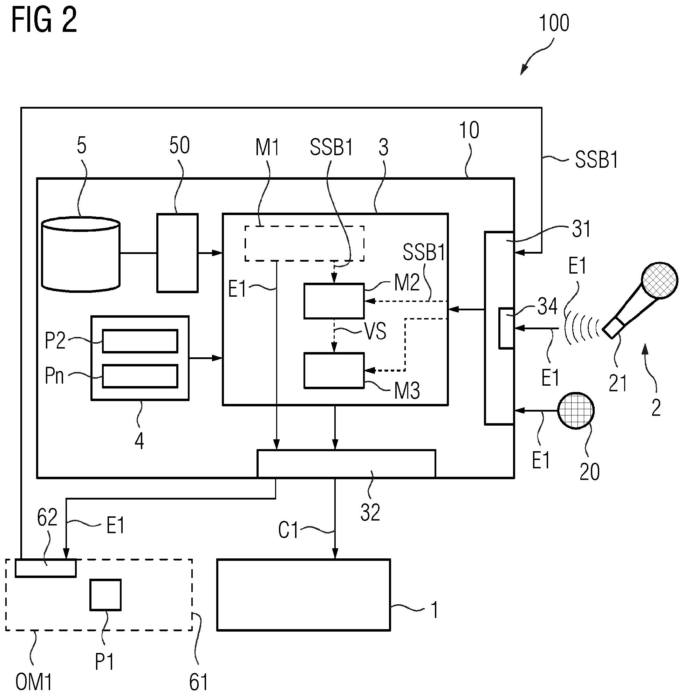

**Figur 2** zeigt schematisch eine funktionale Blockdarstellung eines Systems 100 zur Durchführung einer medizinischen Prozedur an einem Patienten gemäß einer weiteren Ausführungsform.

Die in Figur 2 gezeigte Ausführungsform unterscheidet sich von der in Figur 1 gezeigten Ausführungsform einerseits darin, dass die Funktionalitäten des Moduls M1 zumindest teilweise in ein Online-Modul OM1 ausgelagert sind. Ansonsten bezeichnen gleiche Bezugszeichen gleiche oder funktionsgleiche Komponenten.

Das Online-Modul OM1 kann auf einem Server 61 abgelegt sein, mit dem die Sprachanalysevorrichtung 10 über eine Internetverbindung und eine Schnittstelle 62 des Servers 61 in Datenaustausch treten kann. Die Sprachsteuervorrichtung 10 kann entsprechend dazu ausgebildet sein, dem Online-Modul OM1 das Audiosignal E1 zu übermitteln. Das Online-Modul OM1 kann dazu ausgebildet sein, basierend auf dem Audiosignal E1 einen ersten Sprachbefehl SSB1 und ggf. die dazugehörige Sicherheitsklasse zu ermitteln und der Sprachsteuervorrichtung 10 zurückzugeben. Entsprechend kann das Online-Spracherkennungsmodul OM1 dazu ausgebildet sein, den ersten Computerlinguistik-Algorithmus P1 in einem geeigneten Online-Speicher verfügbar zu machen. Das Online-Modul OM1 kann dabei als zentralisierte Einrichtung aufgefasst werden, welche Spracherkennungsdienste für mehrere, insbesondere lokale Clients bereitstellt (die Sprachsteuervorrichtung 10 kann in diesem Sinne als lokaler Client aufgefasst werden). Die Verwendung eines zentralen Online-Moduls OM1 kann dahingehend vorteilhaft sein, dass leistungsfähigere Algorithmen angewandt und mehr Rechenleistung aufgebracht werden können.

In alternativen Implementierungen kann das Online-Spracherkennungsmodul OM1 auch "lediglich" das erste Sprachanalyseergebnis SAE1 zurückgeben. Das erste Sprachanalyseergebnis kann dann bspw. maschinen-verwertbaren Text enthalten, in den das Audiosignal E1 umgewandelt wurde. Auf Grundlage dessen kann das Modul M1 der Recheneinheit 3 den ersten Sprachbefehl SSB1 identifizieren. Eine solche Ausgestaltung kann dann von Vorteil sein, wenn die Sprachbefehle SSB1 von den Gegebenheiten der medizinischen Vorrichtung 1 abhängen, auf die das Online-Modul OM1 keinen Zugriff hat und/oder für deren Berücksichtigung das Online-Modul OM1 nicht vorbereitet wurde. Man bedient sich dann der Leistungsfähigkeit des Online-Moduls OM1 zur Erstellung eines ersten Sprachanalyseergebnisses, bestimmt die Sprachbefehle aber ansonsten innerhalb der Recheneinheit 3.

Gemäß einer weiteren, nicht gezeigten Abwandlung können umgekehrt aber auch weitere Funktionen der Sprachanalysevorrichtung 10 in einem zentralen Server ausgeführt werden. So ist denkbar, auch den zweiten Computerlinguistik-Algorithmus P2 in einem Online-Modul zu hosten.

Andererseits unterscheidet sich die hier gezeigte von der in Fig. 2 gezeigten Ausführungsform durch eine weitere akustische Eingabevorrichtung 20. Diese dient ebenfalls zum Aufnehmen oder Erfassen des Audiosignals E1. Das Erfassen erfolgt dabei zeitgleich bzw. parallel zu dem Erfassen des Audiosignals E1 mittels Eingabevorrichtung 2. Die Eingabevorrichtung 20 kann ebenfalls als Mikrophon realisiert sein, welches losgelöst von der Eingabevorrichtung 20 direkt in einem Gehäuse der medizinische Vorrichtung 1 oder an anderer Stelle, wie in einem Bedienraum, stationär angeordnet sein kann. Auch Eingabevorrichtung 20 kann in Ausbildungen, wie Eingabevorrichtung 2, zur drahtlosen Datenkommunikation mit Eingang 31 ausgebildet sein. Eingang 31 ist hier ausgebildet, das mittels Eingabevorrichtung 2 erfasste Audiosignal für das Analysieren und Bereitstellen des ersten Sprachanalyseergebnisses SAE1 und das mittels Eingabevorrichtung 20 erfassten Audiosignals für das Analysieren und Bereitstellen des zweiten Sprachanalyseergebnisses SAE2 bereitzustellen. Fehler bei der Signalerfassung, also bei der Registrierung, der Digitalisierung und/oder der Speicherung der Spracheingabe werden so vorteilhaft auf einen Auswertungspfad des Audiosignals beschränkt.

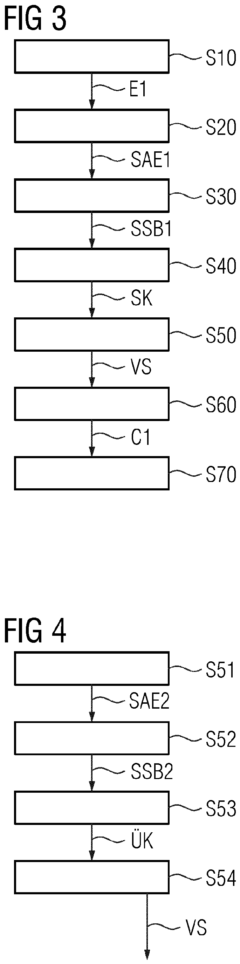

Eine Steuerung der medizinischen Vorrichtung 1 erfolgt in dem in Figur 1 beispielhaft dargestellten System 100 durch ein Verfahren, welches in **Figur 3** beispielhaft als Flussdiagramm dargestellt ist. Die Reihenfolge der Verfahrensschritte ist weder durch die dargestellte Abfolge noch durch die gewählte Nummerierung beschränkt. So kann die Reihenfolge der Schritte ggf. vertauscht werden. Einzelne Schritte können in Ausführungen parallel durchgeführt werden. Wiederum können einzelne Schritte weggelassen werden.

Allgemein ist hierbei vorgesehen, dass die Bedienperson, welche die medizinische Vorrichtung 1 bedient, ein Kommando stimmlich bzw. sprachlich äußert, z.B. indem sie einen Satz wie "Starte Röntgenbildaufnahme" oder "Bringe Patient in Ausgangsposition" spricht, die Eingabevorrichtung 2 und ggf. die Eingabevorrichtung 20 ein zugehöriges Audiosignal E1 erfasst und verarbeitet und die Sprachsteuervorrichtung 10 das erfasste Audiosignal E1 analysiert und ein entsprechendes Steuerkommando C1 zur Betätigung der medizinischen Vorrichtung 1 erzeugt. Ein Vorteil dieses Vorgehens ist, dass die Bedienperson während des Sprechens auch andere Aufgaben erledigen kann, z.B. die Vorbereitung des Patienten. Dies beschleunigt vorteilhaft die Arbeitsabläufe. Ferner kann die medizinische Vorrichtung 1 dadurch zumindest teilweise "berührungslos" gesteuert werden, wodurch die Hygiene an der medizinische Vorrichtung 1 verbessert wird.

Das Verfahren zur Sprachsteuerung der medizinischen Vorrichtung 1 umfasst die Schritte S10 bis S70. Die Schritte werden bevorzugt mit der Sprachsteuervorrichtung 10 ausgeführt. In Schritt S10 erfolgt ein Erfassen eines Audiosignals E1 enthaltend eine auf die Steuerung der Vorrichtung 1 gerichtete Spracheingabe einer Bedienperson mittels der Eingabevorrichtung 2. In Ausführungen erfolgt in Schritt S10 ein gleichzeitiges Erfassen des Audiosignals E1 mit Eingabevorrichtung 20.

Das Audiosignal E1 wird der Sprachsteuervorrichtung 10 über Eingang 31 bereitgestellt. Ein Schritt S20 umfasst ein Analysieren des Audiosignals E1 zum Bereitstellen eines ersten Sprachanalyseergebnisses SAE1. Dazu kann insbesondere das mittels Eingabevorrichtung 2 erfasste Audiosignal bereitgestellt werden. Schritt S20 umfasst also ein Bereitstellen einer zur Steuerung der medizinischen Vorrichtung relevanten sprachlichen Äußerung als erstes Sprachanalyseergebnis SAE1 aus dem Audiosignal E1. Das Erzeugen des Sprachanalyseergebnisses SAE1 kann mehrere Teilschritte umfassen.

Ein Teilschritt kann darauf gerichtet sein, zunächst die im Audiosignal E1 enthaltene Schallinformation in Textinformation umzuwandeln, d.h. ein Transkript zu erzeugen. Ein Teilschritt kann darauf gerichtet sein, eine Tokenisierung des Audiosignals E1 bzw. der Spracheingabe der Bedienperson bzw. des Transkripts T vorzunehmen. Tokenisierung bezeichnet dabei die Segmentierung der Spracheingabe, d.h. den gesprochenen Text in Einheiten der Wort- bzw. Satzebene. Entsprechend kann das erste Sprachanalyseergebnis SAE1 eine erste Tokenisierungsinformation umfassen, welche beispielsweise angibt, ob die Bedienperson einen momentanen Satz zu Ende gesprochen hat oder nicht.

Ein Teilschritt kann darauf gerichtet sein, zusätzlich oder alternativ eine semantische Analyse des Audiosignals E1 bzw. der Spracheingabe der Bedienperson bzw. des Transkripts T auszuführen. Entsprechend kann das erste Sprachanalyseergebnis SAE1 eine erste semantische Information der Spracheingabe der Bedienperson umfassen. Die semantische Analyse ist dabei darauf gerichtet, der Spracheingabe eine Bedeutung zuzuordnen. Dazu kann beispielsweise Wort-weise oder Wortgruppen-weise ein Abgleich mit einer allgemeinen oder für die medizinische Vorrichtung 1 spezifischen Wort- und/oder Sprachbefehlsdatenbank 5 bzw. einer Wort- und/oder Sprachbefehlsbibliothek 50 der medizinischen Vorrichtung 1 bzw. des erfindungsgemäßen Systems 100 erfolgen. Insbesondere können in diesem Schritt bspw. dem Transkript T ein oder mehrere in einer Befehlsbibliothek 50 der medizinischen Vorrichtung 1 enthaltene Aussagen entsprechend verschiedener Sprachbefehle zuordenbar sind. So kann eine auf einen Sprachbefehl gerichtete Nutzerintention erkannt werden.

Die beschriebenen Teilschritte können von einem von Modul M1 umfassten Sprachverständnismodul ausgeführt werden, insbesondere können diese Teilschritte von dem Online-Modul OM1 ausgeführt werden.

In Schritt S30 erfolgt ein Erkennen eines ersten Sprachbefehls SSB1 basierend auf dem ersten Sprachanalyseergebnis SAE1. Hier wird insbesondere die semantische Information repräsentierend eine Nutzerintention genutzt, um einen entsprechenden Sprachbefehl als ersten Sprachbefehl SSB1 zuzuordnen. Anders ausgedrückt, wird der erkannte Sprachbefehl einer von vielen möglichen Befehlsklassen zugeordnet, wobei für eine Befehlsklasse verschiedene Ausdruckweisen, Worte, Wortfolgen oder Wortkombinationen hinterlegt sein können, die die dem Sprachbefehl entsprechende Nutzerintention repräsentieren können. Schritt S30 kann ebenfalls einen Abgleich des ersten Sprachanalyseergebnisses SAE1 mit einer Befehlsdatenbank 5 bzw. Befehlsbibliothek 50 umfassen.

In einem optionalen Schritt S40 erfolgt ein Zuordnen des ersten Sprachbefehls SSB1 zu einer Sicherheitsklasse SK. Hierbei ist erfindungsgemäß mindestens eine Sicherheitsklasse für sicherheitskritische Sprachbefehle vorgesehen, zu welcher der erste Sprachbefehl SSB1 gehört. Dabei kann jeder Sprachbefehl mittels klassischer Lookup-Tabelle oder einer anderen Zuordnungsvorschrift einer Sicherheitsklasse SK zugeordnet sein. Erfindungsgemäß ist nun wenigstens eine der möglichen Sicherheitsklassen für sicherheitskritische Sprachbefehle vorgesehen, deren Ausführung durch die medizinische Vorrichtung 1 spezifischen, insbesondere genormten genormten Sicherheitsanforderungen genügen muss. Insbesondere sind hier Sprachbefehle umfasst, deren Ausführung erstfehlersicher realisiert werden muss.

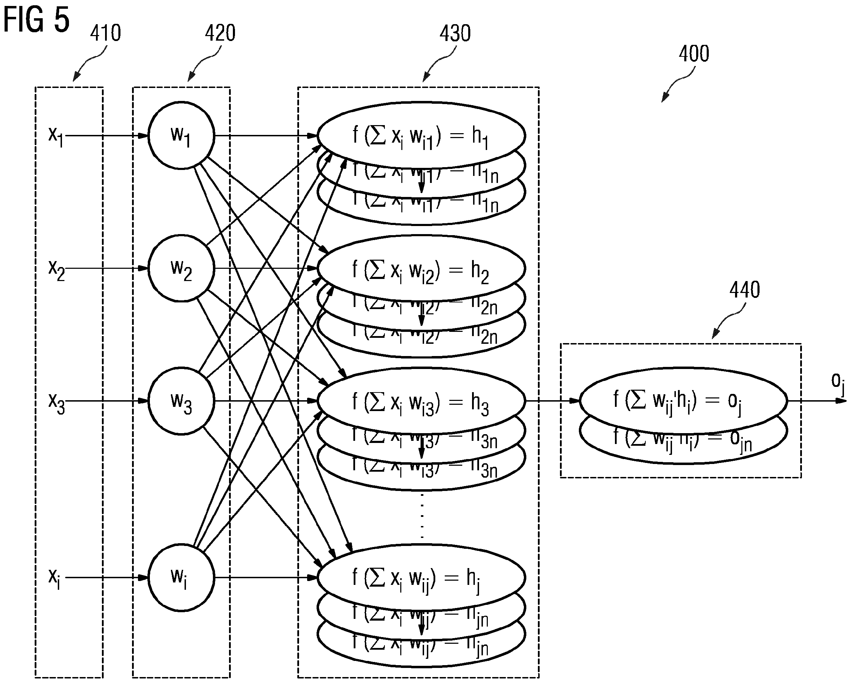

Die Schritte S20 bis S40 können bspw. durch eine Software P1 realisiert werden, die auf dem Datenspeicher 4 abgelegt ist und die Recheneinheit 3, insbesondere Modul M1 oder Online-Modul OM1 zur Durchführung dieser Schritte veranlasst. Die Software P1 kann einen ersten Computerlinguistik-Algorithmus umfassend eine erste trainierte Funktion umfassen, die auf das Audiosignal, insbesondere zum Analysieren des Audiosignals E1, angewandt wird, der mit Bezug zu Figuren 4 und 5 noch näher erläutert wird. Damit realisiert die Erfindung einen klassischen C-Pfad einer CP-Architektur.

In Schritt S50 erfolgt ein Ermitteln eines Verifikationssignals VS zur Bestätigung des ersten Sprachbefehls SSB1. Das Verifikationssignal VS wird basierend auf dem erfassten Audiosignal E1 ermittelt. Für Schritt S50 kann bevorzugt das mittels Eingabevorrichtung 20 erfasste Audiosignal bereitgestellt werden. Das Verifikationssignal VS kann erfindungsgemäß in mehreren Teilschritten ermittelt werden, wie mit Bezug Figur 4 noch näher erläutert wird. Die Teilschritte S51 bis S54 von S50 sind auf ein Analysieren des Audiosignals zum Bereitstellen eines zweiten Sprachanalyseergebnisses SAE2, auf ein Erkennen eines zweiten Sprachbefehls SSB2 der Bedienperson basierend auf dem zweiten Sprachanalyseergebnis und auf ein Vergleichen des ersten und des zweiten Sprachbefehls gerichtet.

Das Ermitteln des Verifikationssignals erfolgt erfindungsgemäß mit dem eigenständigen Modul M2 der Recheneinheit 3. Damit realisiert die Erfindung einen eigenständigen P-Pfad zur Absicherung sicherheitskritischer Sprachbefehle, wobei eine entsprechende Sicherheitsfunktion erfindungsgemäß auf einem mittels Methoden der maschinellen Sprachverarbeitung gewonnenen Sprachbefehls basiert.

Während Schritt S50 auf dem mittels Eingabevorrichtung 20 oder Eingabevorrichtung 2 erfassten Audiosignal ausgeführt wird, werden die Schritte S20 bis S40 auf dem mittels Eingabevorrichtung 2 erfassten Audiosignal ausgeführt.

Schritt S50 kann in Ausführungen parallel, also gleichzeitig zu den Schritten S20 bis S40 ausgeführt werden. Alternativ kann Schritt S50 im Anschluss an die Schritt S20 bis S40 ausgeführt werden. Schritt S50 kann in Ausführungen für jeden Steuerbefehl ausgeführt werden, der in Schritt S40 als erster Sprachbefehl SSB1 identifiziert wurde. In bevorzugten Ausführungen wird Schritt S50 in Abhängigkeit von Schritt S40 ausgeführt, nämlich dann, wenn der erste Sprachbefehl SSB1 durch Zuordnen zu einer Sicherheitsklasse SK umfassend sicherheitskritische Sprachbefehl als solcher, insbesondere als erstfehlersicherer Sprachbefehl identifiziert wurde.

In Schritt S60 erfolgt ein Erzeugen eines Steuersignals C1 zur Steuerung der medizinischen Vorrichtung basierend auf dem Verifikationssignal VS bzw. dem ersten Sprachbefehl SSB1, sofern der erste Sprachbefehl SSB1 in Schritt S50 bestätigt wurde. Das Steuersignal C1 ist dann geeignet, die medizinische Vorrichtung 1 entsprechend dem ersten Sprachbefehl SSB1 zu steuern. Der erste Sprachbefehl SSB1 kann in Form einer Eingangsvariable oder als Teil des Verifikationssignals VS Modul M3 der Recheneinheit 3 (oder einer entsprechenden Software, die beispielsweise auf dem Datenspeicher 4 abgelegt ist) übergeben und wenigstens ein Steuersignal C1 daraus abgeleitet werden. In einem Schritt S70 erfolgt die Eingabe bzw. Übergabe des Steuersignals C1 in die medizinische Vorrichtung 1 über den Ausgang 32.

**Figur 4** zeigt ein Ablaufdiagramm zur Ermittlung eines Verifikationssignals VS für den ersten Sprachbefehl SSB1 in einer Ausführung der Erfindung. Hier erfolgt eine Bestätigung des ersten Sprachbefehls SSB1 ohne weitere Nutzerinteraktion. Mit anderen Worten ermöglicht die Erfindung eine besonders einfache und schnelle Bedienung der medizinischen Vorrichtung 1 mit nur einer initialen Spracheingabe im Sinne des Audiosignals E1.

Die in Figur 5 dargestellten Schritte, deren Reihenfolge durch den Ablauf ebenfalls nicht zwingend vorgegeben ist, können im Rahmen von Schritt S50 aus Figur 3 erfolgen. Dementsprechend umfasst Schritt S50, also das Ermitteln des Verifikationssignals VS, die folgenden Schritte.

Schritt S51 ist auf ein Analysieren des Audiosignals E1 zum Bereitstellen eines zweiten Sprachanalyseergebnisses SAE2 gerichtet und Schritt S52 ist auf ein Erkennen eines zweiten Sprachbefehls SSB2 basierend auf dem zweiten Sprachanalyseergebnis SAE2.

Die Schritte S51 bis S54 werden durch eine Software P2 realisiert, die auf dem Datenspeicher 4 abgelegt ist und die Recheneinheit 3, insbesondere Modul M2 zur Durchführung dieser Schritte veranlasst. Die Software P2 kann einen zweiten Computerlinguistik-Algorithmus P2 umfassend eine zweite trainierte Funktion umfassen, die auch, so wie Software P1 auf das Audiosignal E1, insbesondere zum Analysieren des Audiosignals E1, angewandt wird.

Schritt S51 kann ebenfalls eine Tokenisierung des Audiosignals E1 und/oder eine semantische Analyse des Audiosignals E1 umfassen. Entsprechend kann das zweite Sprachanalyseergebnis SAE2 auch eine zweite Tokenisierungsinformation bzw. eine zweite semantische Information umfassen.

Der zweite Computerlinguistik-Algorithmus P2 kann vorteilhaft eine zweite trainierte Funktion umfassen. Kennzeichnend für die vorliegende Erfindung ist, dass sich die erste trainierte Funktion und die zweite trainierte Funktion voneinander unterscheiden. Insbesondere ist die zweite trainierte Funktion ausgebildet, nur sicherheitskritische, insbesondere erstfehlersicher abzubildende Sprachbefehle in dem Audiosignal E1 zu identifizieren.

Dagegen ist die erste trainierte Funktion ausgebildet, einen sehr breiten Wortschatz bzgl. verschiedenster Spracheingaben einer Bedienperson und insbesondere auch nicht sicherheitskritische Sprachbefehle zu erkennen. Die zweite trainierte Funktion ist in diesem Sinne spezifisch für sicherheitskritische Sprachbefehle ausgebildet. Die zweite trainierte Funktion erkennt also keine nicht sicherheitskritischen Sprachbefehle bzw. allg. Spracheingaben. Insofern ist zumindest Schritt S52 spezifisch für das Erkennen eines sicherheitskritischen Sprachbefehls als zweiten Sprachbefehl SSB2.

Sicherheitskritische Sprachbefehle sind durch eine charakteristische Merkmalskombination, aus bspw. dem Frequenzmuster, der Amplitude, der Modulation, oder dergleichen gekennzeichnet. Sicherheitskritische, insbesondere erstfehlersichere Sprachbefehle weisen zudem insbesondere eine Mindest-Silbenanzahl von drei oder mehr auf. Je größer die Anzahl der Silben eines Sprachbefehls, umso größer einfacher kann er von anderen Sprachbefehlen unterschieden werden. Eine Verwechslungsgefahr zu anderen Sprachbefehlen wird dadurch minimiert.

Die Spezifität der Schritte S51 und S52 wird erreicht, indem die zweite trainierte Funktion anders als die erste trainierte Funktion ausgebildet wird. Ein wesentlicher Unterschied zwischen der ersten und der zweiten trainierten Funktion kann zwischen den jeweils verwendeten Trainingsdaten liegen. Der erste Trainingsdatensatz für die erste trainierte Funktion umfasst einen breiten und vielseitigen Sprachschatz bzgl. verschiedenster Sprachbefehle bzw. allgemeiner Spracheingaben. Der zweite Trainingsdatensatz für die zweite trainierte Funktion ist hingegen auf einen Sprachschatz beschränkt, der auf spezifische, insbesondere phonetisch eindeutige Sprachbefehle gerichtet ist, um sicherheitskritische, insbesondere erstfehlersichere Sprachbefehle eindeutig mit geringer Fehlerrate erkennen zu können. Insofern kann die zweite trainierte Funktion erfindungsgemäß mittels eines kleinen Trainings-Wortschatz (engl. small vocabulary) und im Gegensatz dazu die erste trainierte Funktion mit einem großen Trainings-Wortschatz (engl. large vocabulary) trainiert werden.

Bspw. kann der erste Trainingsdatensatz in Bezug auf zwei Funktionen bzw. Arbeitsschritte der medizinischen Vorrichtung 1 eine Vielzahl von Spracheingaben umfassen. Eine erste Funktion kann ein sicherheitskritisches Starten einer Röntgenbildaufnahme betreffen. Die Eingabedaten des ersten Trainingsdatensatzes können bspw. folgende Spracheingaben umfassen: "New Scan!", "Please, start a new scan!", "Let's do a new acquisition now!", "Will you perform a scan form me?" oder dergleichen umfassen. Die entsprechenden Ausgabedaten des ersten Trainingsdatensatzes beschränken sich auf den einen Sprachbefehl "Perform acquisition!". Die erste trainierte Funktion wird also so trainiert, dass sie alle diese Spracheingaben in dieselbe Befehlsklasse umfassend den Sprachbefehl "Perform acquisition!" klassifiziert. Die erste trainierte Funktion kann dabei auch zur weiteren Verallgemeinerung ausgebildet sein, sodass sie auch bspw. die Spracheingabe "Let's do a new performance for me!" dem o.g. Sprachbefehl zuordnen würde.

Eine zweite Funktion kann bspw. ein nicht sicherheitskritisches Starten eines Ventilators der medizinischen Vorrichtung 1 betreffen. Die Eingabedaten des ersten Trainingsdatensatzes können bspw. folgende Spracheingaben umfassen: "Start fan!", "Switch on the fan!", "OK, activate the fan!" oder dergleichen umfassen. Die entsprechenden Ausgabedaten des ersten Trainingsdatensatzes beschränken sich auf den einen Sprachbefehl "Start fan!". Die erste trainierte Funktion wird ferner so trainiert, dass sie alle diese Spracheingaben in die weitere Befehlsklasse umfassend den Sprachbefehl "Start fan!" klassifiziert.

Entsprechend können die ersten Trainingsdaten eine große Vielzahl weiterer Eingabedaten und Ausgabedaten betreffend weitere und insbesondere beliebige Sprachbefehle und sonstige Eingaben umfassen.

Die zweite trainierte Funktion ist insbesondere ausgebildet, anhand der zweiten Tokenisierungsinformation und/oder der zweiten semantischen Information strukturell und/oder lexikalisch eindeutige Sprachbefehle als sicherheitskritische Sprachbefehle zu identifizieren. Die strukturellen Eigenschaften und/oder lexikalische Eigenschaften des Audiosignals E1 werden in Teilschritt S51 ermittelt. Charakteristische, eindeutige strukturelle und/oder lexikalische Eigenschaften sind dabei mit sicherheitskritischen Sprachbefehlen verknüpft, um diese leicht und verlässlich als solche identifizieren zu können.

In diesem Sinne ist in besonders bevorzugter Ausführung die zweite trainierte Funktion ausgebildet, einen Sprachbefehl mit mindestens drei, bevorzugt vier Silben als sicherheitskritischen Sprachbefehl zu identifizieren, um die Verwechslungsgefahr von sicherheitskritischen Sprachbefehlen zu verringern. Besonders bevorzugt werden also sicherheitskritische Arbeitsschritte der Vorrichtung mit Sprachbefehlen umfassend mindestens drei Silben belegt, besonders bevorzugt mit Sprachbefehlen umfassend fünf bis sieben Silben.

Die zweite trainierte Funktion wird also so trainiert, dass sie nur eine konkrete Spracheingabe als einen sicherheitskritischen Sprachbefehl klassifiziert. Insbesondere kann die zweite trainierte Funktion auf das Erkennen von ein oder mehreren Schlüsselwörtern oder Wortfolgen trainiert werden.

Der zweite Trainingsdatensatz umfasst folglich zu einem sicherheitskritischen Sprachbefehl entsprechen Ausgabedaten genau eine Spracheingabe entsprechend Eingabedaten. Die Spracheingabe wird nun erfindungsgemäß als strukturell, lexikalisch und/oder phonetisch eindeutige Spracheingabe gewählt, um eine Verwechslung mit weiteren Spracheingaben zu vermeiden.

In dem oben genannten Beispiel bezüglich der sicherheitskritischen Funktion der medizinischen Vorrichtung 1, eine Röntgenbildaufnahme zu starten, umfasst der zweite Trainingsdatensatz nun als Spracheingabe "Perform Acquisition" und als Ausgabedaten entsprechend des sicherheitskritischen Sprachbefehls "Perform Acquisition". Besonders bevorzugt kann die Spracheingabe einen Trigger- oder Startbegriff umfassen, bspw. im Sinne von "Siemens, perform Acquisition", wobei Siemens den spezifischen Triggerbegriff darstellt.

Ein alternativer, möglicher Sprachbefehl zum Auslösen von Röntgenstrahlung könnte auch lauten: "Start Scan!". Dieser ist wegen seiner akustischen Ähnlichkeit zu "Start Fan" aber sehr einfach zu verwechseln. Ein weiterer alternativer Sprachbefehl könnte "New Scan!" lauten. Dieser ist jedoch mit zwei Silben auch leicht verwechselbar und könnte als Teil einer allgemeinen Spracheingabe miterfasst und fälschlich als Sprachbefehl erkannt werden, bspw. als Bestandteil der Spracheingabe "When the patient is in we're doing a new scan immediately".

Der Sprachbefehl "Perform Acquisition" hingegen ist sowohl aufgrund seiner akustischen Merkmale als auch wegen seiner sieben Silben nur schwer verwechselbar und daher als sicherheitskritischer Sprachbefehl besonders gut geeignet.

Die trainierten Funktionen selbst können sich erfindungsgemäß ebenfalls unterscheiden, speziell in der Ausbildung der Verifikationsfunktion bzw. der Klassifizierungsfunktion. Die erste trainierte Funktion weist eine große Anzahl Kategorien entsprechend einer Vielzahl verschiedener Sprachbefehle auf. Die zweite trainierte Funktion hingegen ist auf eine geringere Menge Kategorien entsprechend einer kleinen, insbesondere für die medizinische Vorrichtung spezifischer sicherheitskritischer Sprachbefehle beschränkt. Durch den Einsatz unterschiedlicher trainierter Funktionstypen reduziert die Erfindung das Risiko für das Auftreten ähnlicher systematischer Fehler bei der Sprachbefehlserkennung durch die erste trainierte Funktion und die zweite trainierte Funktion.

Identifiziert die zweite trainierte Funktion in Schritt S52 einen Sprachbefehl als zweiten Sprachbefehl SSB2 aus dem zweiten Sprachanalyseergebnis SAE2, handelt es sich also per se um einen sicherheitskritischen Sprachbefehl.

Durch die verschiedenartige Ausbildung der ersten und zweiten trainierten Funktionen wird in dieser Ausführung das Risiko einer Ausführung eines fehlerhaft im Modul M1 erkannten ersten Sprachbefehls SSB1 minimiert, da systematische Sprachbefehlserkennungsfehler der ersten trainierten Funktion mit großer Wahrscheinlichkeit nicht von der zweiten trainierten Funktion wiederholt werden.

In einem Schritt S53 wird nun die Übereinstimmung zwischen dem ersten und dem zweiten Sprachbefehl SSB1, SSB2 anhand eines Übereinstimmungskriteriums ÜK geprüft. Dabei wird ein Übereinstimmungsmaß zwischen den beiden Sprachbefehlen mit einem für den ersten und/oder zweiten Sprachbefehl SSB1/SSB2 spezifischen und vorab festgelegten Schwellwert verglichen. Der Schwellwert bzw. das erforderliche Ähnlichkeitsmaß kann von Sprachbefehl zu Sprachbefehl unterschiedlich groß ausfallen. Schwellwerte von Sprachbefehlen einer hohen Sicherheitsstufe, also insbesondere erstfehlersicher abzubildenden Sprachbefehlen, sind erfindungsgemäß größer ausgebildet als von Sprachbefehlen einer niedrigeren Sicherheitsstufe.

Ergibt der Prüfschritt S53, dass der erste und der zweite Sprachbefehl SSB1, SSB2 ein bei oder oberhalb des Schwellwertes liegendes Übereinstimmungsmaß aufweisen, ist der erste Sprachbefehl SSB1 bestätigt.

Modul M2 der Sprachsteuervorrichtung 10 erzeugt in Schritt S54 weiter ein Verifikationssignal VS basierend auf dem Prüfergebnis aus Schritt S53. Das Verifikationssignal VS wird im Folgenden an Modul M3 entsprechend der Steuereinheit der Sprachsteuervorrichtung 10 übertragen, welche (in Schritt S60 in Fig. 3) das Steuersignal C1 entsprechend dem ersten, mittels Verifikationssignal VS bestätigten Sprachbefehl SSB1 generiert.

Im oben genannten Beispiel des sicherheitskritischen Sprachbefehls zum Starten einer Röntgenbildaufnahme würde die medizinische Vorrichtung also nur dann eine Röntgenstrahlung auslösen, wenn die Bedienperson als Spracheingabe "Perform Acquisition" sagt.

**Figur 6** zeigt ein künstliches neuronales Netz 400, wie es in Verfahren gemäß der Figuren 3 bis 4 zum Einsatz kommen kann. Insbesondere kann es sich bei dem gezeigten neuronalen Netz 400 um die erste oder zweite trainierte Funktion des ersten bzw. des zweiten Computerlinguistik-Algorithmus P1, P2 handeln. Das neuronale Netz 400 antwortet auf Eingabewerte zu einer Vielzahl von Eingangsknoten $x_i$ 410 die angewendet werden, um eine oder eine Vielzahl von Ausgaben $o_j$ zu erzeugen. Das neuronale Netz 400 lernt in diesem Ausführungsbeispiel, indem es die Gewichtungsfaktoren $w_i$ (weights) der einzelnen Knoten basierend auf Trainingsdaten anpasst. Mögliche Eingabewerte der Eingangsknoten $x_i$ 410 können bspw. Spracheingaben bzw. Audiosignale eines ersten oder eines zweiten Trainingsdatensatzes sein. Das neuronale Netz 400 gewichtet 420 die Eingabewerte 410 basierend auf dem Lernprozess. Die Ausgabewerte 440 des neuronalen Netzes 400 entsprechen in Ausführungen einem ersten bzw. einem zweiten Sprachbefehl SSB1, SSB2. Die Ausgabewerte 440 des neuronalen Netzes können in anderen Ausführungen auch eine Angabe zu einer Sicherheitsklasse SK des ersten Sprachbefehls SSB1 oder ein Ergebnis einer Überprüfung eines Übereinstimmungskriteriums zwischen erstem und zweitem Sprachbefehl SSB1, SSB2 umfassen. Die Ausgabe 440 kann über einen einzelnen oder eine Vielzahl von Ausgabeknoten $o_j$ erfolgen.

Das künstliche neuronale Netz 400 umfasst bevorzugt eine versteckte Schicht 430, die eine Vielzahl von Knoten $h_j$ umfasst. Es können mehrere versteckte Schichten $h_{jn}$ vorgesehen sein, wobei eine versteckte Schicht 430 Ausgabewerte einer anderen (versteckten) Schicht 430 als Eingabewerte verwendet. Die Knoten einer versteckten Schicht 430 verrichten mathematische Operationen. Ein Ausgabewert eines Knotens $h_j$ entspricht dabei einer nicht-linearen Funktion f seiner Eingabewerte $x_i$ und der Gewichtungsfaktoren $w_i$. Nach dem Erhalt von Eingabewerten $x_i$, führt ein Knoten $h_j$ eine Summierung einer mit den Gewichtungsfaktoren $w_i$ gewichteten Multiplikation jedes Eingabewerts $x_i$ durch, wie durch folgende Funktion bestimmt:

$$h_j = f\left(\sum_i x_i \cdot w_{ij}\right)$$

Insbesondere wird ein Ausgabewert eines Knotens $h_j$ als Funktion f einer Knoten-Aktivierung, bspw. eine Sigmoidalfunktion oder eine lineare Rampenfunktion gebildet. Die Ausgabewerte $h_j$ werden an den bzw. die Ausgabeknoten $o_j$ übertragen. Erneut wird eine Summierung einer gewichteten Multiplikation jedes Ausgabewertes $h_j$ als Funktion der Knoten-Aktivierung f berechnet:

$$o_j = f\left(\sum_i h_i \cdot w'_{ij}\right)$$

Das hier gezeigte neuronale Netz 400 ist ein vorwärts gerichtetes (engl. feedforward) neuronales Netz, wie es bevorzugt für den ersten Computerlinguistik-Algorithmus P1 zum Einsatz kommt, bei dem alle Knoten 430 die Ausgabewerte einer vorherigen Schicht in Form ihrer gewichteten Summe als Eingabewerte verarbeiten. Insbesondere für den zweiten Computerlinguistik-Algorithmus können erfindungsgemäß andere neuronale Netztypen zum Einsatz kommen, bspw. rückkoppelndes, bspw. selbstlernendes (engl. recurrent) neuronales Netz, bei denen ein Ausgabewert eines Knotens $h_j$ gleichzeitig

auch sein eigener Eingabewert sein kann.

Das neuronale Netz 400 wird bevorzugt mittels einer Methode des überwachten Lernens trainiert, um Muster zu erkennen. Eine bekannte Vorgehensweise ist die Back-Propagation, die für alle Ausführungsbeispiele der Erfindung angewandt werden kann. Während des Trainings wird das neuronale Netz 400 auf Trainings-Eingabewerten angewandt und muss entsprechende, vorher bekannte Ausgabewerte erzeugen. Iterativ werden mittlere quadratische Fehler (mean square error - "MSE") zwischen berechneten und erwarteten Ausgabewerten berechnet und einzelne Gewichtungsfaktoren 420 so lange angepasst, bis die Abweichung zwischen berechneten und erwarteten Ausgabewerten unterhalb einer vorbestimmten Schwelle liegt.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

Die Erfindung ermöglicht die Nutzung von Computerlinguistik-Algorithmen umfassend neuronale Netze zur Spracherkennung und Ableitung von Sprachbefehlen in sicherheitsrelevanten Anwendungen, die insbesondere erstfehlersicher ausgebildet sein müssen. In Tests konnte belegt werden, dass die beschriebene Lösung zuverlässiger als klassisch implementierte Spracherkennungsalgorithmen ohne Sicherheitsfunktion sind. Die Erfindung ist in Ausführungen durch eine Skalierbarkeit hinsichtlich Benutzerfreundlichkeit und/oder Sicherheit (Fehleraufdeckung) gekennzeichnet. Dies kann einerseits genutzt werden, um Sprachbefehle unterschiedlicher Sicherheitsstufen jeweils angemessenen abzusichern. Andererseits ist es möglich, mit der Zeit die Bedienbarkeit zu verbessern, wenn Erfahrungen vorliegen, dass eine Sprachbefehlserkennung bzw. Sprachbefehlsverifikation in einer definierten Zielumgebung gewünschten Sicherheitsvorgaben entspricht und eine ausreichende Zuverlässigkeit nachgewiesen werden konnte. Alternativ ist es möglich, die Sicherheit zu erhöhen, wenn erkannt wurde, dass bspw. aufgrund spezieller Umgebungsbedingungen (bspw. Hintergrundgeräusche) eine zuverlässige Erkennung der per Spracheingabe übermittelten Kommandos nicht ausreichend gut funktioniert.

Die Skalierbarkeit ermöglicht allgemein eine graduelle Änderung der sicherheitstechnischen Belastung zwischen konventionell implementierten Anteilen und über Methoden des Maschinellen Lernens implementierten Anteilen. Hierdurch kann ein Migrationspfad in Richtung der sicherheitstechnisch belastbaren Nutzung von Computerlinguistik-Algorithmen umfassend neuronale Netze geschaffen werden.

Abschließend sei darauf hingewiesen, dass die erfindungsgemäße Ausbildung eines Verifikationsmechanismus für mittels Spracherkennung abgeleitete Steuerbefehle in Bezug auf eine falsche Interpretation erkannter Kommandos (einer eine Nutzerintention umfassende Spracheingabe wird ein falscher Sprachbefehl zugeordnet) oder in Bezug auf eine unzeitige, also eine zu langsame Erkennung von Sprachkommandos angewendet werden kann. Erfindungsgemäß kann jedoch ein Fehler, bei dem mit dem ersten Computerlinguistik-Algorithmus in einer Spracheingabe trotz enthaltener Nutzerintention kein Sprachbefehl erkannt wird, nicht abgesichert werden. Daher ist die vorliegende Erfindung nicht geeignet, bspw. eine Not-Stopp Funktion abzusichern.

**Patentansprüche**

1. Verfahren zur Sprachsteuerung einer medizinischen Vorrichtung (1) mit den Schritten:

   - Erfassen (S10) eines Audiosignals (E1) enthaltend eine auf die Steuerung der Vorrichtung gerichtete Spracheingabe einer Bedienperson;
   - Analysieren (S20) des Audiosignals zum Bereitstellen eines ersten Sprachanalyseergebnisses (SAE1),
   - Erkennen (S30) eines ersten Sprachbefehls (SSB1) der Bedienperson basierend auf dem ersten Sprachanalyseergebnis,
   - Ermitteln (S50) eines Verifikationssignals (VS) für den ersten Sprachbefehl, umfassend:

     - Analysieren (S51) des Audiosignals zum Bereitstellen eines zweiten Sprachanalyseergebnisses (SAE2),
     - Erkennen (S52) eines zweiten Sprachbefehls (SSB2) der Bedienperson basierend auf dem zweiten Sprachanalyseergebnis,
     - Vergleichen (S53) des ersten und des zweiten Sprachbefehls,

   wobei das Verifikationssignal den ersten Sprachbefehl bestätigt, wenn der erste Sprachbefehl und der zweite Sprachbefehl ein Übereinstimmungskriterium (ÜK) erfüllen,
   - Erzeugen (S60) eines Steuersignals (C1) zur Steuerung der medizinischen Vorrichtung basierend dem Verifikationssignal, sofern der erste Sprachbefehl anhand des Verifikationssignals bestätigt wurde, wobei das Steuersignal geeignet ist, die medizinische Vorrichtung entsprechend dem ersten Sprachbefehl zu steuern, und
   - Eingabe (S70) des Steuersignals in die medizinische Vorrichtung.

2. Verfahren nach Anspruch 1, wobei

- das Analysieren (S20) zum Bereitstellen des ersten Sprachanalyseergebnisses ein Anwenden eines ersten Computerlinguistik-Algorithmus (P1) umfassend eine erste trainierte Funktion auf das Audiosignal umfasst, und
- das Analysieren (S51) zum Bereitstellen eines zweiten Sprachanalyseergebnisses ein Anwenden eines zweiten Computerlinguistik-Algorithmus (P2) umfassend eine zweite trainierte Funktion auf das Audiosignal umfasst,

wobei sich die erste trainierte Funktion und die zweite trainierte Funktion voneinander unterscheiden.

3. Verfahren nach Anspruch 2, wobei die zweite trainierte Funktion ausgebildet ist, nur sicherheitskritische Sprachbefehle zu erkennen.

4. Verfahren nach einem der Ansprüche 1 bis 3, wobei das Analysieren des Audiosignals ein Tokenisieren zur Segmentierung von Buchstaben, Wörtern und/oder Sätzen innerhalb des Audiosignals mittels des ersten und des zweiten Computerlinguistik-Algorithmus umfassen und der erste und der zweite Sprachbefehl basierend auf dem eine erste Tokenisierungsinformation umfassenden ersten Sprachanalyseergebnis und dem eine zweite Tokenisierungsinformation umfassenden zweiten Sprachanalyseergebnis erkannt werden.

5. Verfahren nach einem der Ansprüche 1 bis 4, wobei das Analysieren des Audiosignals eine semantische Analyse des Audiosignals mittels des ersten und des zweiten Computerlinguistik-Algorithmus umfassen und der erste und der zweite Sprachbefehl basierend auf dem eine erste semantische Information umfassenden ersten Sprachanalyseergebnis und dem eine zweite semantische Information umfassenden zweiten Sprachanalyseergebnis identifiziert werden.

6. Verfahren nach Anspruch 3 in Verbindung mit einem der Ansprüche 4 oder 5, wobei die zweite trainierte Funktion ausgebildet ist, anhand der zweiten Tokenisierungsinformation und/oder der zweiten semantischen Information strukturell und/oder lexikalisch eindeutige Sprachbefehle als sicherheitskritische Sprachbefehle zu identifizieren.

7. Verfahren nach einem der Ansprüche 5 oder 6, wobei die zweite trainierte Funktion ausgebildet ist, einen Sprachbefehl mit mindestens drei Silben als sicherheitskritischen Sprachbefehl zu identifizieren.

8. Verfahren nach einem der Ansprüche 2 bis 7, ferner umfassend die Schritte:

- Klassifizieren (S40) des ersten Sprachbefehls in eine Sicherheitsklasse aus einer Vielzahl von Sicherheitsklassen, wobei wenigstens eine der mehreren Sicherheitsklassen für sicherheitskritische Sprachbefehle vorgesehen ist,
- Ermitteln eines Verifikationssignals für den ersten Sprachbefehl, wenn dieser der Sicherheitsklasse für sicherheitskritische Sprachbefehle zugeordnet wurde.

9. Verfahren nach einem der vorherigen Ansprüche, wobei das Erfassen des Audiosignals folgendes umfasst:

- Erfassen des Audiosignals mittels einer ersten Eingabevorrichtung (2) und einer zweiten Eingabevorrichtung (20), wobei sich die erste und die zweite Eingabevorrichtung voneinander unterscheiden,
- Bereitstellen des mittels der ersten Eingabevorrichtung erfassten Audiosignals für das Analysieren und Bereitstellen des ersten Sprachanalyseergebnisses, und
- Bereitstellen des mittels der zweiten Eingabevorrichtung erfassten Audiosignals für das Analysieren und Bereitstellen des zweiten Sprachanalyseergebnisses.

10. Sprachsteuervorrichtung (10) zur Sprachsteuerung einer medizinischen Vorrichtung (1), umfassend

- eine Schnittstelle (31) zum Erfassen eines Audiosignals (E1) enthaltend eine auf die Steuerung der Vorrichtung gerichtete Spracheingabe einer Bedienperson;
- ein erste Auswerteeinheit (M1), die ausgebildet ist,

- das Audiosignal zu analysieren und ein erstes Sprachanalyseergebnis (SAE1) bereitzustellen,
- einen ersten Sprachbefehl (SSB1) basierend auf dem ersten Sprachanalyseergebnis zu erkennen,

- eine zweite Auswerteeinheit (M2), die ausgebildet ist,

- ein Verifikationssignal (VS) für den ersten Sprachbefehl zu ermitteln durch

- Analysieren des Audiosignals (E1) zum Bereitstellen eines zweiten Sprachanalyseergebnisses (SAE2),
- Erkennen eines zweiten Sprachbefehls (SSB2) der Bedienperson basierend auf dem zweiten Sprachanalyseergebnis,
- Vergleichen des ersten und des zweiten Sprachbefehls,

wobei das Verifikationssignal den ersten Sprachbefehl bestätigt, wenn der erste Sprachbefehl und der zweite Sprachbefehl ein Übereinstimmungskriterium (ÜK) erfüllen,

- eine Steuereinheit (M3), die ausgebildet ist,

- ein Steuersignal (C1) zur Steuerung der medizinischen Vorrichtung basierend auf dem Verifikationssignal zu erzeugen, sofern der erste Sprachbefehl anhand des Verifikationssignals bestätigt wurde, wobei das Steuersignal geeignet ist, die medizinische Vorrichtung entsprechend dem ersten Sprachbefehl zu steuern, und

- eine Schnittstelle (32) zur Eingabe des Steuersignals in die medizinische Vorrichtung.

11. Sprachsteuervorrichtung nach Anspruch 10, wobei die erste und die zweite Auswerteeinheit als voneinander getrennte Recheneinheiten ausgebildet sind.

12. Sprachsteuervorrichtung nach einem der Ansprüche 10 oder 11, wobei die Schnittstelle (31) eine erste und eine zweite Eingabevorrichtung (2, 20) zum gleichzeitigen Erfassen des Audiosignals umfasst.

13. Medizinisches System (1) umfassend:

- eine Sprachsteuervorrichtung (10) nach einem der Ansprüche 10 bis 12, und
- eine medizinische Vorrichtung (1) zur Durchführung einer medizinischen Prozedur.

14. Computerprogrammprodukt, welches ein Programm umfasst und direkt in einen Speicher einer programmierbaren Recheneinheit ladbar ist, mit Programmmitteln, um ein Verfahren nach den Ansprüchen 1 bis 9 auszuführen, wenn das Programm ausgeführt wird.

15. Computerlesbares Speichermedium, auf welchem lesbare und ausführbare Programmabschnitte gespeichert sind, um alle Schritte der Verfahren nach einem der Ansprüche 1 bis 9 auszuführen, wenn die Programmabschnitte ausgeführt werden.

FIG 1

FIG 2

## FIG 3

```
┌─────────────────────┐
│                     │~ S10
└─────────────────────┘
           │
           │~ E1
           ▼
┌─────────────────────┐
│                     │~ S20
└─────────────────────┘
           │
           │~ SAE1
           ▼
┌─────────────────────┐
│                     │~ S30
└─────────────────────┘
           │
           │~ SSB1
           ▼
┌─────────────────────┐
│                     │~ S40
└─────────────────────┘
           │
           │~ SK
           ▼
┌─────────────────────┐
│                     │~ S50
└─────────────────────┘
           │
           │~ VS
           ▼
┌─────────────────────┐
│                     │~ S60
└─────────────────────┘
           │
           │~ C1
           ▼
┌─────────────────────┐
│                     │~ S70
└─────────────────────┘
```

## FIG 4

```
┌─────────────────────┐
│                     │~ S51
└─────────────────────┘
           │
           │~ SAE2
           ▼
┌─────────────────────┐
│                     │~ S52
└─────────────────────┘
           │
           │~ SSB2
           ▼
┌─────────────────────┐
│                     │~ S53
└─────────────────────┘
           │
           │~ ÜK
           ▼
┌─────────────────────┐
│                     │~ S54
└─────────────────────┘
           │
           │~ VS
           ▼
```

EP 4 156 179 A1

FIG 5

Europäisches
Patentamt

European
Patent Office

Office européen
des brevets

**EUROPÄISCHER RECHERCHENBERICHT**

Nummer der Anmeldung

**EP 21 19 8655**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2014/195247 A1 (PARKINSON CHRISTOPHER [US] ET AL) 10. Juli 2014 (2014-07-10) | 1-6,8, 10,11, 13-15 | INV. G10L15/32 A61B17/00 |
| Y | * Absätze [0025], [0049] - [0052], [0054] - [0062], [0075] - [0079] * * Abbildungen 4, 7 * ----- | 7 | ADD. G10L15/22 |
| X | US 2011/063429 A1 (CONTOLINI MATTEO [US] ET AL) 17. März 2011 (2011-03-17) * Absätze [0004], [0008], [0013], [0030], [0033] - [0036], [0054] * ----- | 1,9,10, 12,14,15 | |
| X | US 2014/006027 A1 (KIM JUHEE [KR] ET AL) 2. Januar 2014 (2014-01-02) * Absätze [0104] - [0114] * * Abbildung 4 * ----- | 1,10,14, 15 | |
| X | US 2018/357999 A1 (LEE KYUNG CHUL [KR] ET AL) 13. Dezember 2018 (2018-12-13) * Absätze [0013], [0042] - [0048] * ----- | 1,10,14, 15 | |
| X | WO 2021/008035 A1 (PING AN TECH SHENZHEN CO LTD [CN]) 21. Januar 2021 (2021-01-21) * Absätze [0080] - [0084] * ----- | 1,10,14, 15 | RECHERCHIERTE SACHGEBIETE (IPC) G10L A61B G06F |
| X | WO 2017/012242 A1 (BAIDU ONLINE NETWORK TECH CO LTD [CN]) 26. Januar 2017 (2017-01-26) * Seite 3, Zeile 27 - Seite 4, Zeile 32 * * Seite 7, Zeile 14 - Zeile 24 * ----- | 1,10,14, 15 | |
| Y | US 2015/154953 A1 (BAPAT OJAS A [US] ET AL) 4. Juni 2015 (2015-06-04) * Absatz [0029] * ----- | 7 | |
| Y | US 2020/286465 A1 (WANG JUN [CN] ET AL) 10. September 2020 (2020-09-10) * Absatz [0074] * ----- | 7 | |

Der vorliegende Recherchenbericht wurde für alle Patentansprüche erstellt

| Recherchenort | Abschlußdatum der Recherche | Prüfer |
|---|---|---|
| **München** | **2. März 2022** | **Geißler, Christian** |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..............................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 19 8655

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-03-2022

| Im Recherchenbericht angeführtes Patentdokument | Datum der Veröffentlichung | Mitglied(er) der Patentfamilie | Datum der Veröffentlichung |
|---|---|---|---|
| US 2014195247 A1 | 10-07-2014 | CN 105009202 A | 28-10-2015 |
| | | EP 2941769 A1 | 11-11-2015 |
| | | JP 6420769 B2 | 07-11-2018 |
| | | JP 2016505897 A | 25-02-2016 |
| | | US 2014195247 A1 | 10-07-2014 |
| | | WO 2014107413 A1 | 10-07-2014 |
| US 2011063429 A1 | 17-03-2011 | CA 2714816 A1 | 16-03-2011 |
| | | EP 2298178 A1 | 23-03-2011 |
| | | JP 5330344 B2 | 30-10-2013 |
| | | JP 2011087920 A | 06-05-2011 |
| | | US 2011063429 A1 | 17-03-2011 |
| US 2014006027 A1 | 02-01-2014 | CN 103533154 A | 22-01-2014 |
| | | EP 2680257 A1 | 01-01-2014 |
| | | JP 5956384 B2 | 27-07-2016 |
| | | JP 2014010456 A | 20-01-2014 |
| | | KR 20140001711 A | 07-01-2014 |
| | | US 2014006027 A1 | 02-01-2014 |
| | | WO 2014003329 A1 | 03-01-2014 |
| US 2018357999 A1 | 13-12-2018 | CN 109003626 A | 14-12-2018 |
| | | KR 20180133703 A | 17-12-2018 |
| | | US 2018357999 A1 | 13-12-2018 |
| WO 2021008035 A1 | 21-01-2021 | CN 110517673 A | 29-11-2019 |
| | | WO 2021008035 A1 | 21-01-2021 |
| WO 2017012242 A1 | 26-01-2017 | CN 105139849 A | 09-12-2015 |
| | | WO 2017012242 A1 | 26-01-2017 |
| US 2015154953 A1 | 04-06-2015 | KEINE | |
| US 2020286465 A1 | 10-09-2020 | CN 108305617 A | 20-07-2018 |
| | | CN 110444193 A | 12-11-2019 |
| | | CN 110444195 A | 12-11-2019 |
| | | EP 3748629 A1 | 09-12-2020 |
| | | JP 2021512362 A | 13-05-2021 |
| | | US 2020286465 A1 | 10-09-2020 |
| | | WO 2019149108 A1 | 08-08-2019 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006045719 B4